# EUROPEAN PATENT APPLICATION

(11) **EP 2 998 406 A1**
(43) Date of publication of application: **23.03.2016**
(21) Application number: 14797327.5
(22) Date of filing: 09.05.2014
(51) Int. Cl.: C12Q 1/04, C12N 5/09, C12M 1/34

(54) **METHOD FOR MONITORING METASTASIS OF CANCER CELLS USING CELLS CULTURED IN THREE-DIMENSIONAL COLLAGEN ENVIRONMENT**

(30) Priority: 14.05.2013 KR 20130054262
(71) Applicant: Medicinal Bioconvergence Research Center, Suwon-si, Gyeonggi-do 443-270 (KR)
(72) Inventor: LEE, Jung Weon, Seoul 151-015 (KR); KIM, Sunghoon, Seoul 135-859 (KR); LEE, Mi-Sook, Seoul 156-751 (KR)
(74) Representative: Pearson, Samuel John
(86) International application number: PCT/KR2014/004146
(87) International publication number: WO 2014/185661

(57) **Abstract**

The present invention relates to a method for monitoring migration, invasion, and metastasis of cancer cells by observing the shape of cancer cells cultured in a three-dimensional environment and measuring the activity, expression, and changes in expression sites of proteins associated with invadopodia formation and metastasis, and the degradation of an extracellular matrix; and to a method for screening a cancer metastasis inhibitor. More specifically, it was verified that the reduction in c-Jun phosphorylation induced the increase in snail1 and the decrease in cortactin expression in the cells cultured in a three-dimensional environment and the expression regulation relations between the proteins were identical to those in breast cancer tissues obtained from patients. In addition, it was verified that, when breast cancer cells in a three-dimensional collagen gel environment were treated with a JNK inhibitor, the shape of the cells became longer; the contact region of the cells and the extracellular matrix became flattened and thinner; the migration of cancer cells was decreased; and the changes in protein expression was observed, such as the increase in TGFβ1 expression, the increases in smad2 and smad3 expression and activity, the increase in snail1 expression, the decrease in cortactin expression, and the resulting decrease in invadopodia formation. In addition, in a three-dimensional collagen gel environment, MT1-MMP besides the cortactin can be used as a marker of invadopodia, and it was verified that the inhibition of JNK led to the decrease in cortactin expression and the increase in snail1 expression, badly influenced the site and role of MT1-MMP to inhibit the formation of invadopodia, and inhibited the degrading activity of a collagen gel substrate. Thus, the present invention can be used as a method for monitoring migration, invasion, metastasis, and the degree of metastasis of cancer cells and a method for screening a cancer metastasis inhibitor, and will be useful as one of screening methods capable of creating low-cost, high-efficient added value at the time of pre-clinical tests required for drug development.

## Description

### BACKGROUND OF THE INVENTION

### 1. Field of the Invention

The present invention relates to a method for monitoring migration, invasion, and metastasis of cancer cells by observing the shape of cancer cells cultured in a three-dimensional environment and measuring the activity, expression, and changes in the expression sites of proteins associated with metastasis, and the degradation of an extracellular matrix; and to a method for screening a cancer metastasis inhibitor.

### 2. Description of the Related Art

The reason that cancer is lethal to a patient is metastasis. Metastasis is the process of the dissemination of cells from the primary tumor, by which cancer cells can be spread wide. The said primary tumor can be developed by various genetic reasons of a host, which makes the treatment of each individual difficult. However, metastasis is the general phenomenon observed in every cancer, so that it is an important target of therapeutic intervention. The metastatic cancer cells leave the primary tumor, pass through basement membrane, and then invade into other tissues or organs. The basement membrane is the supporting layer under epithelium that plays a role as an extracellular matrix (ECM) protein network. Once cancer cells are disseminated from the primary tumor, they travel through blood stream. The metastatic cancer cells form the invasive protrusions like invadopodia that is the suitable structure for cancer cells to invade blood vessels or lymphatic ducts with decomposing ECM and penetrating stromal layer. Invadopodia, the invasive protrusion wherein F-actin is accumulated, is located on the contact area of cells and matrix and has the capability of matrix degradation. This is the kind of structure where the intracellular signaling factors, protein degradation, cell adhesion, cytoskeleton, and membrane-trafficking pathway are gathered.

To treat cancer, chemotherapy using anticancer agents or radio-therapy is the widely used conventional method. To develop an anticancer agent for chemotherapy, countless efforts and costs have been consumed. Nevertheless, it is still not very successful to develop a satisfactory anticancer agent. The pre-clinical tests are also essential to verify the effect of drug candidates for the development of an anticancer agent. High recurrence rate or death rate of cancer is attributed to the metastasis of cancer that survives the surgical operation. Therefore, it would be an innovation in the development of an anticancer agent if the whole process of cancer metastasis is understood at the molecular level, or if a method and clue to control the mechanism of metastasis is established or the analysis system thereof is established. Many researchers are looking for such a model system that is close to a human body environment or a patient body environment for the pre-clinical tests, and the most representative example is the cell culture in a three-dimensional extracellular matrix environment, tissue remodeling, or a humanized animal system.

Despite the tissues and organs that form a living body are three-dimensional structures, the test methods to understand cell formation, cell function, and pathological characteristics have depended on a two-dimensional cell culture method or a two-dimensional animal model system. The conventional two-dimensional cell culture method-based studies made important theoretical progresses. However, since the cell morphology and interaction between cells or between cells and ECM in a real human body are different, animal models have been used to modify and complement the accuracy of tests. Animal models are genetically different systems from human, suggesting that the tests with animal models might bring inappropriate results for human in the aspects of cancer treatment and drug reaction and autoimmune disease, etc. Besides, it takes a long time and high costs to establish an animal model and to analyze with the animal system, limiting the experiment in realizing a target model. But, three-dimensional (3D) cell culture can overcome the limit of the conventional cell culture and can make up the weakness of the animal model. 3D cell culture method can provide an artificial control system by including or excluding a specific intracellular or microenvironmental factor therein, and is useful for verifying various hypotheses because the cell shape and signaling activity are closer to those of *in vivo,* and is advantageous in performing different experiments at the same time. It is also easy with 3D cell culture to observe the three-dimensional cell shape in a specific environment under microscope in real-time.

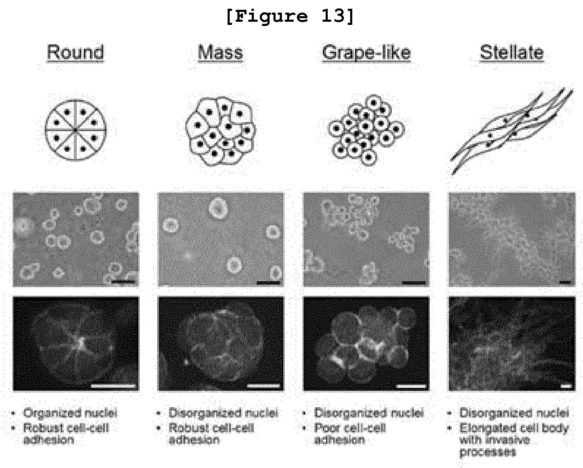

Cells produce and store ECM proteins that form basement membrane. The basement membrane is a thin layer of specific ECM, which supports the epidermal layer and the endothelial layer and is composed of such proteins that connect cell and matrix as laminin, collagen, fibronectin, and entactin. These proteins play an important role in the regulatory mechanisms of cellular behavior including cell migration, adhesion, wound-healing, and scattering, etc. A three-dimensional scaffold can be constructed in a lab with ECM. The three-dimensional scaffold plays a role as a temporary support for cells in a specific environment and then is eventually embodied *in vivo.* Scaffold has been widely used in the field of tissue engineering. Unlike the two-dimensional cell monolayer, this scaffold is a three-dimensional support having the original cell geometry. Most of the natural scaffolds being used these days are natural hydrogel such as type 1 collagen, type IV collagen, laminin, fibronectin, or hyaluronic acid. The said natural hydrogel is physically weak but can provide a biological environment to cells. The collagen hydrogel scaffold is often used for the construction of a three-dimensional organotypic breast cancer cell model. However, this collagen hydrogel cannot copy the real stiffness of the real tissue cells and cancer cells. In a three-dimensional culture, cells display various shapes according to the type, concentration, and hardness of the extracellular matrix (see Figure 13).

Breast cancer can be divided largely into 4 groups, and some of which are very rare. Sometimes, one type of breast cancer can display combination type. Ductal carcinoma in situ (DCIS) is the most common non-invasive breast cancer. In DCIS, cancer cells exist in ducts and yet not passing through the duct wall enveloping the breast tissue, suggesting that the cancer cells are not spread yet. About 20% of breast cancer patients are DCIS patients. Once diagnosed with DCIS, the patient needs to be treated early because it can spread out other breast tissues soon. Lobular carcinoma in situ is the cancer developed in lobular, which does not belong to the actual cancer family. Invasive ductal carcinoma (IDC) is the most common type of breast cancer. IDC starts from mammary duct of the breast, breaks through the mammary duct, and then grows in adipose tissue of the breast. At this time, IDC cells can migrate to other organs through lymphatic system and blood vessels. About 80% of breast cancer patients are IDC patients. Invasive lobular carcinoma starts at lobules. Like IDC, this cancer is also metastatic. About 10% of invasive breast cancer patients are ILC patients. Diagnosis of invasive lobular carcinoma (ILC) is more difficult than diagnosis of invasive ductal carcinoma (IDC).

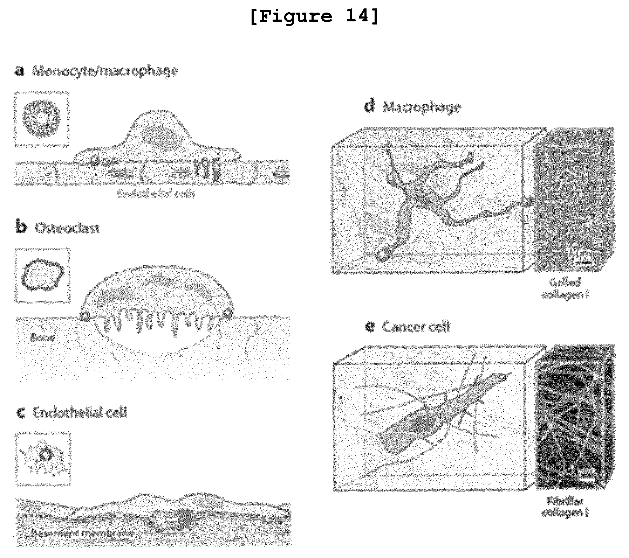

Invadopodia are the actin-rich protrusions observed on the cell membrane. Invadopodia are synthesized from the synthesis of actin core structure and can degrade the extracellular matrix by the accumulation of matrix metalloproteinase. Invadopodia have the function of metastasis and are mostly found in metastatic cancer cells. Invadopodia have a very similar shape to podosome in normal cells such as macrophages, monocytes, and osteoblasts where they can pass through the tissue barrier. In invadopodia (or invadosome), cortactin, tyrosine kinase, and such matrix metalloproteinases MT1-MMP are consolidated and coexist with actin. Unlike in a two-dimensional environment, the cells in a three-dimensional environment can produce invadopodia by changing the shape, cytoskeleton and contacts with matrix (see Figure 14).

JNK [c-Jun N-terminal kinase] is one of MAP (mitogen-activated protein) kinases and is activated by various steps and stimuli. In cancer cells, JNK induces apoptosis or increases cell survival and proliferation, indicating it is involved in both sides of cancer development. For example, the inhibition of JNK activity in some cancer cases could suppress the proliferation of cells or induce apoptosis. JNK activity and c-Jun phosphorylation are also necessary in the transformation induced by ras, the carcinogenic protein.

Snail1 is one of transcription factors which can be up-regulated in relation to epithelial mesenchymal transition (EMT). Snail1 binds to E-box element in E-cadherin gene promoter region and as a result it inhibits transcription and cell-cell adhesion, leading to EMT. Snail1 is induced by TGFβ in various cell lines and regulates the expression of EMT related proteins, and regulates various cell functions including proliferation and apoptosis. The expression of snail1 is increased by TGFβ signal activated by collagen in PDAC (pancreatic ductal adenocarcinoma). Snail1 regulates the process of extracellular fibrosis, but collagen is generated during the process and the produced collagen increases snail1 expression again, resulting in the increase of fibrosis. Snail1 and twist are accumulated in the leading edge of the growing mammary buds, and are accordingly involved in mammary epithelial branching.

The present inventors tried to develop a novel method for monitoring metastasis of cancer cells cultured in a three-dimensional extracellular matrix environment. In the course of study, the inventors confirmed the decrease of c-Jun phosphorylation by JNK activity inhibition, the activation of smad proteins relating to TGFβ1 signaling, the increase of snail1 expression, and the decrease of cortactin expression when the breast cancer cell line MDA-MB-231 was cultured in a three-dimensional collagen gel environment or extracellular acidity was raised or hypoxia was induced by reducing the intracellular oxygen concentration. The present inventors additionally confirmed that such interactions between molecules were identical to those in breast cancer tissues obtained from patients. In addition, when JNK inhibitor was treated to the cells, the cell shape became longer and the contact region of the cells and the extracellular matrix became flattened, cancer cell migration was decreased, and the changes in protein expression were observed such as the increase of snail1 expression, the decrease of cortactin expression, and the inhibition of invadopodia formation thereby. At this time, the inhibition of c-Jun phosphorylation induced the increase of the expressions and phosphorylations of TGFβ1, smad2, and smad3, suggesting that the transcription of snail1 was promoted by the interaction between smad2 and smad4 in the snail1 promoter region, so that snail1 was up-regulated. The inventors further confirmed that MT1-MMP could be used as another invadopodia marker in a three-dimensional collagen gel environment and the inhibition of JNK could increase snail1 expression. The decrease of cortactin expression by snail1 had a negative effect on the location and role of MT1-MMP, resulting in the inhibition of invadopodia formation and the inhibition of the degradation of collagen matrix surrounding invadopodia. By verifying the above, the present inventors completed this invention.

### SUMMARY OF THE INVENTION

It is an object of the present invention to provide a method for monitoring cancer cell migration, invasion, metastasis, and the degree of metastasis.

It is another object of the present invention to provide a method for screening a cancer metastasis inhibitor.

To achieve the above objects, the present invention provides a method for monitoring cancer cell migration, invasion, metastasis, and the degree of metastasis, comprising the following step:
1) culturing cancer cells in a culture vessel in a three-dimensional environment surrounded by extracellular matrix; and
2) measuring the changes in the shape of cancer cells cultured in step 1), and the activity, expression, and changes in expression sites of the proteins associated with invadopodia formation/degradation, migration, invasion, and metastasis, and the degradation of extracellular matrix.

The present invention also provides a method for screening a cancer metastasis inhibitor comprising the following steps:
1) culturing cancer cells in a culture vessel in a three-dimensional environment surrounded by extracellular matrix;
2) treating test samples to the cancer cells of step 1);
3) measuring the activity, expression, and changes in expression sites of the proteins associated with invadopodia formation/degradation, migration, invasion, and metastasis, and the degradation of extracellular matrix; and
4) selecting the test sample that is confirmed to inhibit the formation of invadopodia or inhibit the activity and expression of the invadopodia marker protein or the metastasis associated protein or to have the negative effect on the expression sites of those proteins or on the degradation of extracellular matrix.

### ADVANTAGEOUS EFFECT

The present invention can be used as a method for monitoring the effect of extracellular microenvironment on various cell functions by regulating the extracellular microenvironment in a three-dimensional culture that can copy *in vivo* environment, a method for monitoring cancer cell migration, invasion, metastasis, and the degree of metastasis by imaging the invadopodia formation in cancer cells cultured in a three-dimensional collagen gel environment, and a method for screening a cancer metastasis inhibitor. The present invention can also be useful as one of screening methods capable of creating low-cost, high-efficient added value at the time of pre-clinical tests required for drug development.

### BRIEF DESCRIPTION OF THE DRAWINGS

The application of the preferred embodiments of the present invention is best understood with reference to the accompanying drawings, wherein:
Figure 1A ∼ Figure 1D are diagrams illustrating the changes of c-Jun phosphorylation and snail1 and cortactin expressions in various microenvironments in MDA-MB-231 cultured in a three-dimensional collagen gel environment.
Figure 1E is a diagram illustrating the changes of c-Jun phosphorylation and snail1 and cortactin expressions in breast cancer tissues.
Figures 2A and 2B are diagrams illustrating the changes of the cell shape of MDA-MB-231 by the treatment of JNK inhibitor in a three-dimensional collagen gel environment and the ratio of width (the shortest distance that goes through the nucleus) to length (the longest distance that goes through the nucleus) in the transformed cell line.
   Control: control, and
   SP600125: JNK inhibitor.
Figure 2C is a diagram illustrating the changes of mRNA and protein expressions by the treatment of JNK inhibitor over the time in a three-dimensional collagen gel environment.
   P: positive control
   SP: JNK inhibitor SP600125.
Figure 2D is a diagram illustrating the changes of the protein expression and phosphorylation by the treatment of JNK inhibitor in a three-dimensional collagen gel environment.
   SP: JNK inhibitor SP600125.
Figure 2E is a diagram illustrating the decrease of cortactin expression by the treatment of JNK inhibitor in a three-dimensional collagen gel environment where GFP fluorescent protein itself or GFP conjugated cortactin protein was expressed, confirmed by the observation of the cell shape wherein the shape became longer in the cells expressing GFP but the shape was not getting longer in the cells expressing GFP-cortactin.
   C: control,
   SP: JNK inhibitor SP600125, and
   GFP: green fluorescent protein.
Figures 2F and 2G are diagrams illustrating the suppression of cell shape changes or cell migration induced by the treatment of JNK inhibitor in a three-dimensional collagen gel environment, compared with the control, confirmed by time-lapse imaging.
   Control(Con): control, and
   SP600125(SP): JNK inhibitor.
Figure 3A is a diagram illustrating the changes of cell shape by the treatment of JNK inhibitor in the cells cultured in a three-dimensional matrigel environment.
   Control: control, and
   SP600125: JNK inhibitor.
Figure 3B is a diagram illustrating the changes of snail1 expression by the treatment of JNK inhibitor in the cells cultured in a three-dimensional matrigel environment, matrigel and collagen gel mixture, or collagen gel environment.
   Matri: matrigel,
   M/C: matrigel and collagen gel mixture, and
   Col I: type I collagen.
Figure 3C is a diagram illustrating that the changes of c-Jun phosphorylation and the protein expression by the treatment of JNK inhibitor in a three-dimensional collagen gel environment were JNK inhibitor dose-dependent.
   SP: JNK inhibitor SP600125.
Figure 3D is a diagram illustrating the changes of protein expression by the treatment of p38 or Erk inhibitor in a three-dimensional collagen gel environment.
   Con: control,
   SP: JNK inhibitor SP600125,
   SB: p38 inhibitor SB203580, and
   U0126: Erk inhibitor.
Figures 4A ∼ 4D are diagrams illustrating that the decrease of numbers and level of co-expression sites of cortactin and actin by the treatment of JNK inhibitor in the group with crowded cells and in the group with less cells in a three-dimensional collagen gel environment.
   Control: control, and
   SP600125: JNK inhibitor.
Figures 4E ∼ 4J are diagrams illustrating the decrease of invadopodia formation and the changes of cell shape according to the treatment of JNK inhibitor in the various breast cancer cell lines cultured in a three-dimensional collagen gel environment.
   Control, Cont: control, and
   SP600125, SP: JNK inhibitor.

Figure 4K is a diagram illustrating the degradation of type 1 collagen induced by JNK inhibitor in the breast cancer cell line cultured in a three-dimensional collagen gel environment.
Figure 5 is a diagram illustrating the decrease of cortactin expression in cell membrane and the indynamic changes of cell shape by JNK inhibitor in a three-dimensional collagen gel environment.
   Control: control, and
   SP600125: JNK inhibitor.
Figure 6A is a diagram illustrating the AP-1 binding domains in the snail1 gene promoter region and the E-box binding elements in the cortactin gene promoter region.
Figures 6B ∼ 6D are diagrams illustrating the results of ChIP (chromatin immunoprecipitation) with the whole cell lysate (B) cultured in a three-dimensional collagen gel environment using anti-pS⁶³c-Jun antibody (C) and anti-snail1 antibody (D).
   WCL: whole cell lysate,
   SP: JNK inhibitor SP600125,
   -: control not treated with SP600125, and
   +: SP600125 treated group.
Figures 6E and 6F are diagrams illustrating the changes of the expressions of cortactin and snail1 mRNA over the time after the treatment with the mRNA synthesis inhibitor actinomycin D (ActD) alone or together with JNK inhibitor.
Figure 6G is a diagram illustrating the changes of the expressions of cortactin and snail1 protein over the time after the treatment with the protein synthesis inhibitor cyclohexamide (CHX) alone or together with JNK inhibitor.
Figure 6H is a diagram illustrating the formation of snail1 and cortactin promoter protein-DNA complex by the treatment of JNK inhibitor in the breast cancer cell line cultured in a three-dimensional collagen gel environment.
Figure 7A is a diagram illustrating the changes of smad2 and smad3 mRNA expressions after the culture in a three-dimensional collagen gel environment for 5 days.
Figure 7B is a diagram illustrating the changes of smad2 and smad3 protein expressions after the culture in a three-dimensional collagen gel environment for 5 days
Figures 7C and 7D are diagrams illustrating the changes of smad2 and smad3 expressions and phosphorylations in the cells cultured in a three-dimensional collagen gel environment, according to the treatment of JNK inhibitor or the changes of extracellular pH condition.
   Control: control, and
   SP600125: JNK inhibitor.
Figure 7E is a diagram illustrating the changes of cortactin and snail1 expressions according to the treatment with smad2, smad3, and smad4 shRNAs in a three-dimensional collagen gel environment.
Figure 7F is a diagram illustrating the result of ChIP performed to investigate whether or not the expression of smad2 and smad4 dependent snail1 protein could be changed at translation level in the presence or absence of JNK inhibitor in a three-dimensional collagen gel environment.
Figure 8A is a diagram illustrating the changes of snail1 and cortactin expressions caused by dominant negative JNK1 in a three-dimensional collagen gel environment.
Figure 8B is a diagram illustrating the decrease of the region stained by actin and the changes of cell shape caused by dominant negative JNK1 in a three-dimensional collagen gel environment.
Figure 8C is a diagram illustrating the changes of mRNA expression by the treatment of JNK1 siRNA in a three-dimensional collagen gel environment.
Figure 8D is a diagram illustrating the changes of protein expression by the treatment of JNK1 siRNA in a three-dimensional collagen gel environment.
Figure 8E is a diagram illustrating the changes of cell shape and the decrease of actin-rich region in the cells injected with JNK1 siRNA (marked by arrow) in a three-dimensional collagen gel environment.
Figure 9A is a diagram illustrating the changes of snail1 protein expression when it was over-expressed in a three-dimensional collagen gel environment.
Figures 9B and 9C are diagrams illustrating the decrease of cortactin site under the over-expression of snail1 protein in a three-dimensional collagen gel environment.
Figures 9D and 9E are diagrams illustrating the changes of cortactin mRNA and protein expressions by the treatment of JNK inhibitor when treated with snail1 siRNA in a three-dimensional collagen gel environment.
   siCon: control siRNA, and
   siSnail1: snail1 siRNA.
Figure 10A is a diagram illustrating that the blank suggesting the degradation of collagen stained by green (white arrow) was not observed as much as in the control (yellow arrow) when treated with JNK inhibitor in a three-dimensional collagen gel environment.
Figure 10B is a diagram illustrating that the location of the expression of MT1-MMP, another invadopodia marker, was identical to that of the actin in a three-dimensional collagen gel environment.
Figures 10C and 10D are diagrams illustrating the changes of MT1-MMP expression and the location approaching the nucleus, compared with the control wherein the expression site is in the edge of cell, according to the treatment of JNK inhibitor in a three-dimensional collagen gel environment.
   Control: control, and
   SP600125: JNK inhibitor.
Figures 11A and 11B are diagrams illustrating the changes of cortactin and MT1-MMP expressions, the expression sites thereof, and the dynamic of site changing, according to the treatment of JNK inhibitor in a three-dimensional collagen gel environment.
   Control: control, and
   SP600125: JNK inhibitor.
Figures 12A and 12B are diagrams illustrating the suppression of MT1-MMP function and the inhibition of type I collagen matrix degradation according to the treatment of JNK inhibitor in the breast cancer cell line cultured in a three-dimensional collagen gel environment.
   Control: control, and
   SP600125: JNK inhibitor.
Figure 13 is a diagram illustrating the morphology of the breast cancer cell colony in a three-dimensional culture environment.
Figures 14A ∼ 14C are diagrams illustrating the shape of invadosome generated during the two-dimensional culture.
Figures 14D and 14E are diagrams illustrating the shape of invadosome generated during the three-dimensional culture.

### DESCRIPTION OF THE PREFERRED EMBODIMENTS

Hereinafter, the present invention is described in detail.

The term used in this invention "invadopodia" indicates the region wherein actin and cortactin can be expressed at the same time. Actin is polymerized and strengthened in the protrusion of cell membrane by the action of cortactin, where the matrix metalloprotease is accumulated to degrade extracellular matrix (ECM). In invadopodia, various proteins such as cortactin, gelsolin, vinculin, talin, and paxillin are gathered together, so that various signaling activities are happening there for actin-reconstruction so as to allow cancer cells to degrade matrix.

The present invention provides a method for monitoring cancer cell migration, invasion, metastasis, and the degree of metastasis, comprising the following step:
1) culturing cancer cells in a culture vessel in a three-dimensional environment surrounded by extracellular matrix; and
2) measuring the changes in the shape of cancer cells cultured in step 1), and the activity, expression, and changes in expression sites of the proteins associated with invadopodia formation/degradation, migration, invasion, and metastasis, and the degradation of extracellular matrix.

The cancer is preferably a metastatic cancer or a metastasis inducible cancer, which is preferably selected from the group consisting of breast cancer, liver cancer, stomach cancer, colon cancer, bone cancer, pancreatic cancer, head/neck cancer, uterine cancer, ovarian cancer, rectal cancer, esophageal cancer, small bowel neoplasm, anal cancer, colon carcinoma, fallopian tube carcinoma, endometrial carcinoma, uterine cervical carcinoma, vaginal carcinoma, vulva carcinoma, Hodgkin's disease, prostatic cancer, bladder cancer, kidney cancer, ureter cancer, renal cell carcinoma, renal pelvic cancer, and central nervous system tumor. In a preferred embodiment of the present invention, the cancer is preferably breast cancer, but not always limited thereto.

The cell culture in step 1) is preferably performed under the regulation of cell culture period, cell number (density), extracellular pH, or extracellular oxygen level, but not always limited thereto.

The culture vessel of step 1) is preferably made of one of those materials selected from the group consisting of polydimethylsiloxane (PDMS), polymethylmethacrylate (PMMA), polyacrylates, polycarbonates, polycyclic olefins, polyimides, and polyurethanes, and polydimethylsiloxane was preferably selected in a preferred embodiment of the invention, but not always limited thereto.

For the three-dimensional culture environment in step 1), a natural hydrogel well known to those in the art, which is exemplified by collagen, laminin, fibronectin, or hyaluronic acid, can be used. At this time, the collagen is preferably type 1 collagen, but not always limited thereto. The concentration of the said type I collagen is preferably 1 ∼ 5 mg/mℓ, more preferably 2 ∼ 4 mg/mℓ, and most preferably 2.5 ∼ 3 mg/mℓ. The collagen is preferably prepared as a neutral, but not always limited thereto.

The cell culture in step 1) is performed with 1 x 10⁴ ∼ 2 x 10⁸ cells/mℓ in the culture medium containing the said natural material, and more preferably with 1 x 10⁵ ∼ 2 x 10⁷ cells/mℓ, and most preferably with 1 x 10⁶ ∼ 2 x 10⁶ cells/mℓ, but not always limited thereto.

The change in the shape of the cells in step 2) is characterized by being longer and the contact region of cell and extracellular matrix (ECM) preferably becomes simply flat and the formation of invadopodia is preferably confirmed therein, but not always limited thereto.

The expression site of the metastasis associated protein in step 2) is preferably changed from cell membrane to cytoplasm or around the nucleus, but not always limited thereto, and the changes of the location into other regions except cell membrane are also included.

The change in the activity of the metastasis associated protein in step 2) is preferably characterized by the increase of c-Jun phosphorylation, and the change of the expression is preferably characterized by the decrease of cortactin and the increase of snail1, but not always limited thereto. Herein, the target protein for the investigation of changes in the activity and expression includes all of those proteins that are located in invadopodia and play an important role in the functions of invadopodia.

The cell migration and invasion in step 2) are preferably confirmed by measuring the degradation of collagen gel matrix, and more precisely by investigating the changes of collagen gel matrix degrading activity, but not always limited thereto.

The measurement of the activity and expression of the protein in step 2) is preferably performed by the method selected from the group consisting of Western blotting, RT-PCR, real-time PCR, immunofluorescence, ChIP (chromatin immunoprecipitation), EMSA (Electrophoric Mobility Shift Assay), or ECM degrading activity assay using DQ™-collagen type I, in a preferred embodiment of the invention, but not always limited thereto.

The present invention also provides a method for screening a cancer metastasis inhibitor comprising the following steps:
1) culturing cancer cells in a culture vessel in a three-dimensional environment surrounded by extracellular matrix;
2) treating test samples to the cancer cells of step 1);
3) measuring the activity, expression, and changes in expression sites of the proteins associated with invadopodia formation/degradation, migration, invasion, and metastasis, and the degradation of extracellular matrix; and
4) selecting the test sample that is confirmed to inhibit the formation of invadopodia or inhibit the activity and expression of the invadopodia marker protein or the metastasis associated protein or to have the negative effect on the expression sites of those proteins or on the degradation of extracellular matrix.

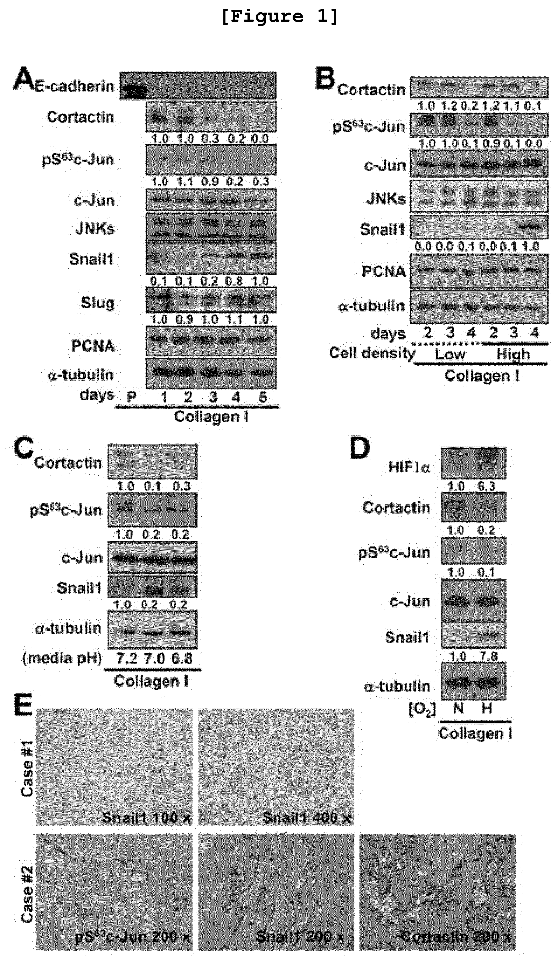

In a preferred embodiment of the present invention, the inventors confirmed the decrease of c-Jun phosphorylation, the decrease of cortactin protein expression, and the increase of snail1 protein expression in the breast cancer cell line MDA-MB-231 cultured in a three-dimensional collagen gel environment (see Figure 1A). The identical results were observed when the microenvironment surrounding the cells was changed, for example cell density or extracellular acidity was changed or hypoxia was induced (see Figures 1B ∼ 1D). In the human breast cancer cell tissues, it was confirmed the decrease of c-Jun phosphorylation and the increase of cortactin expression and the overall expression of snail1 (see Figure 1E). Therefore, it was verified that the changes in the activity and expression of the above proteins in the breast cancer cell line cultured in a three-dimensional collagen gel environment were attributed to the microenvironment.

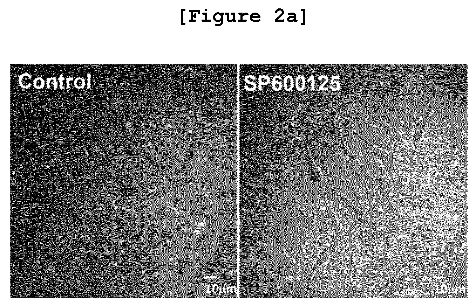

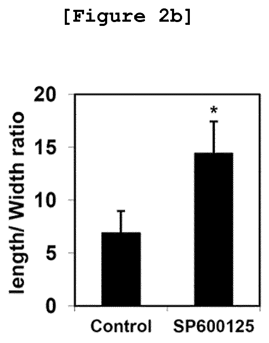

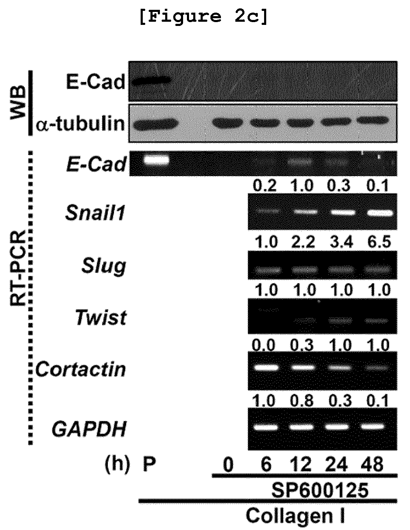

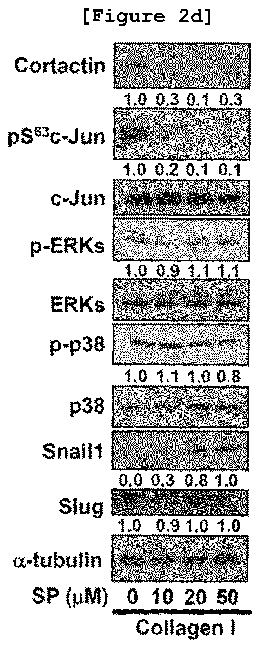

When the breast cancer cell line cultured in a three-dimensional collagen gel environment was treated with JNK inhibitor, the shape of the cells became longer and the contact region of the cells and the extracellular matrix became flattened and thinner, compared with the control (see Figures 2A and 2B). At this time, snail1 and twist mRNA were up-regulated but cortactin mRNA was down-regulated. These increase and decrease were identical at the protein levels. However, the phosphorylations of p38 and ERK were not changed (see Figures 2C and 2D). Therefore, it was confirmed that JNK inhibition induced specifically snail1 expression.

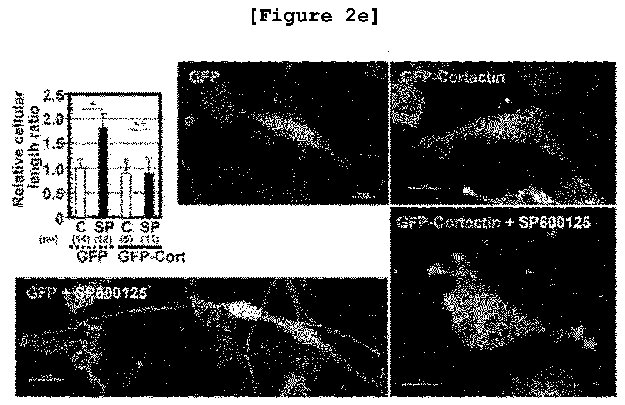

It was also confirmed that the expression of cortactin was suppressed and accordingly cell migration was decreased when the breast cancer cell line cultured in a three-dimensional collagen gel environment was treated with JNK inhibitor (see Figures 2E ∼ 2G).

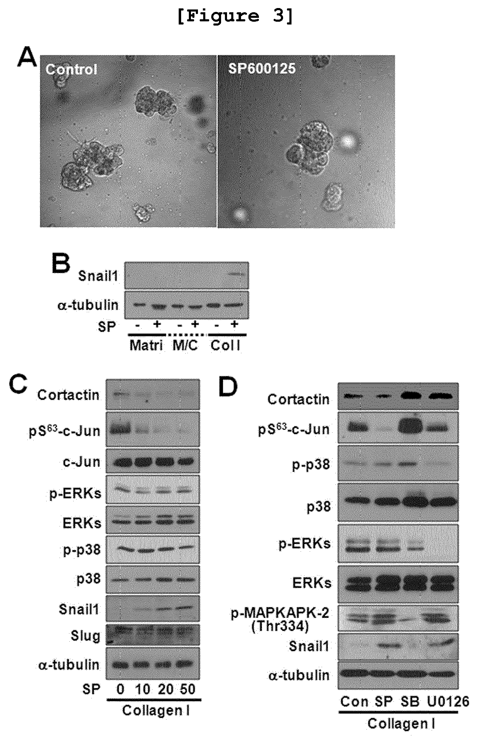

When the beast cancer cell line cultured in a three-dimensional matrigel environment was treated with JNK inhibitor, the changes in the cell shape and the increase of snail1 protein expression were not observed (see Figures 3A and 3B). The changes in the protein expression by p38 or ERK inhibitor were confirmed but the changes in the protein expression by JNK inhibitor were not confirmed (see Figure 3D).

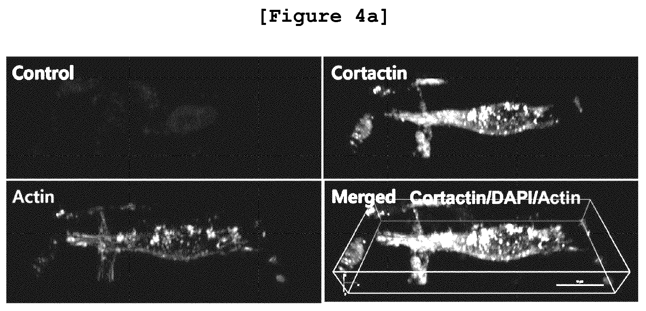

It was also confirmed that the treatment of JNK inhibitor to the breast cancer cell line cultured in a three-dimensional collagen gel environment caused the decrease of cortactin expression (Figure 3C) and the reduction of the region of cortactin and actin interaction, resulting in the decrease of invadopodia formation (see Figures 4A ∼ 4D).

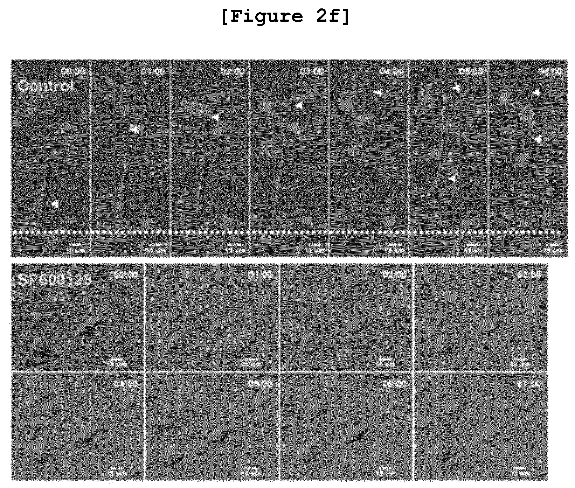

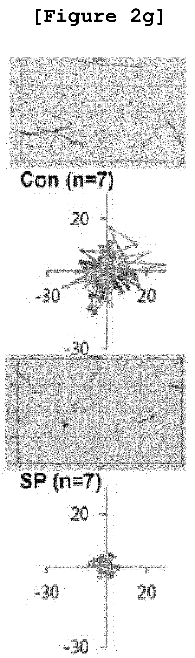

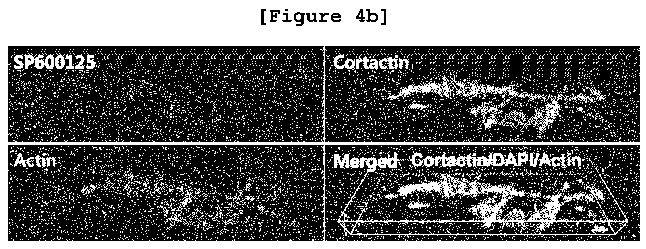

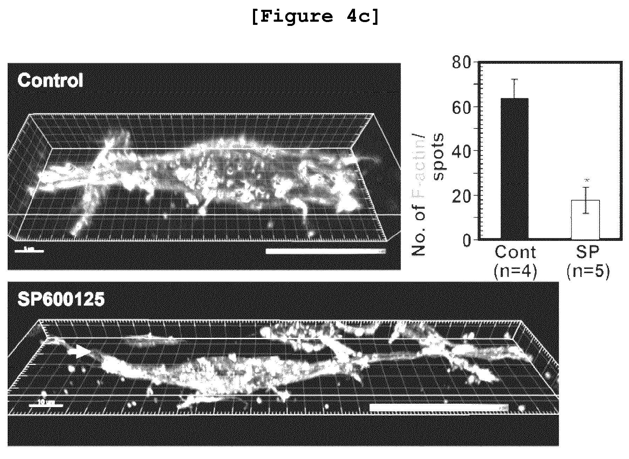

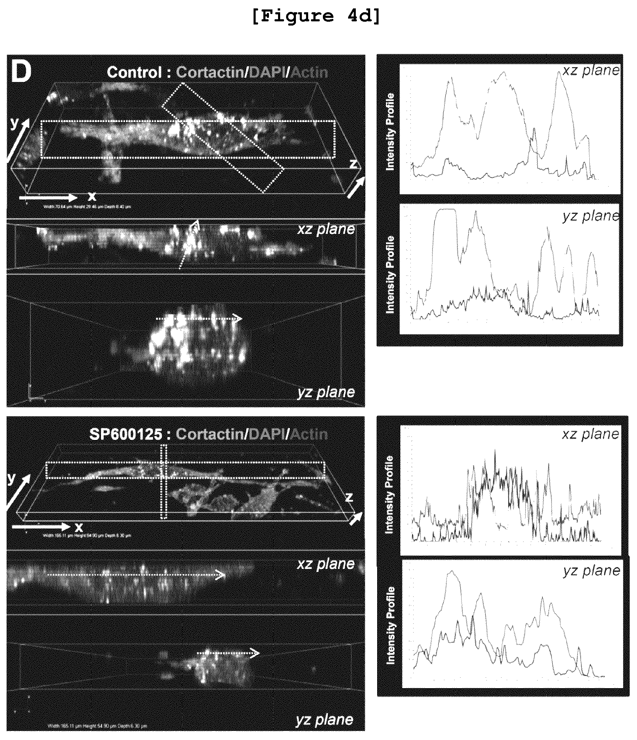

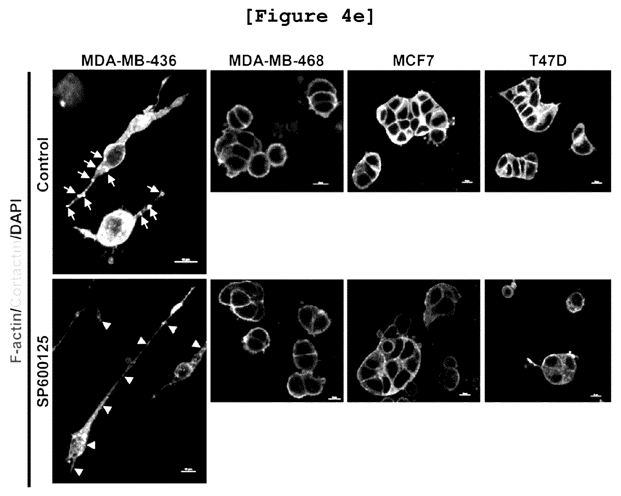

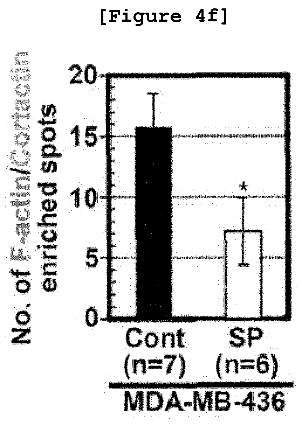

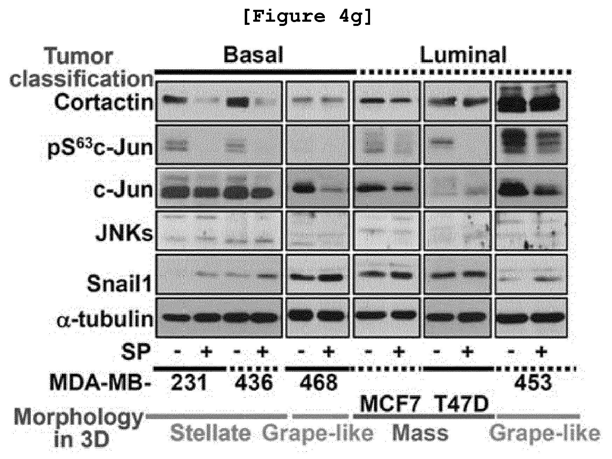

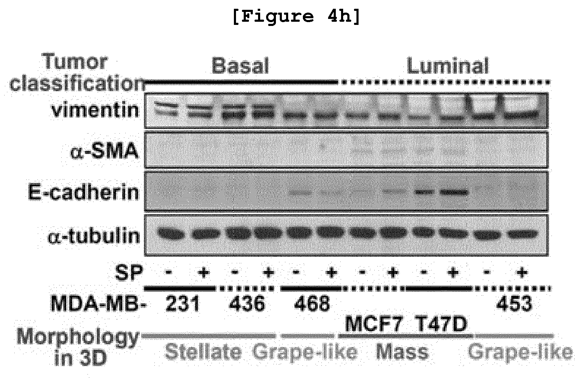

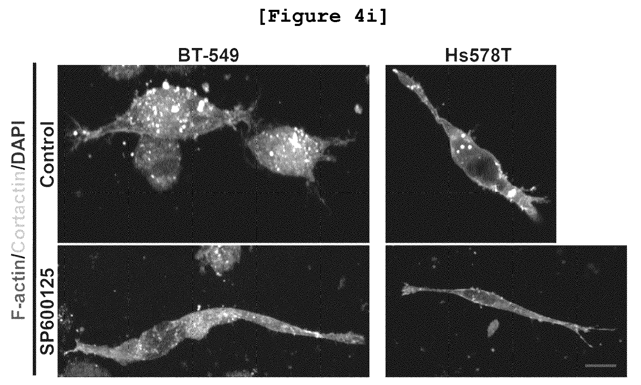

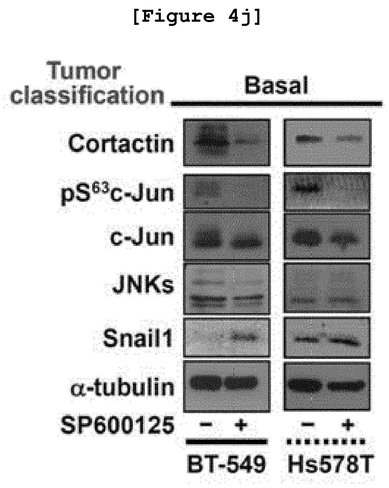

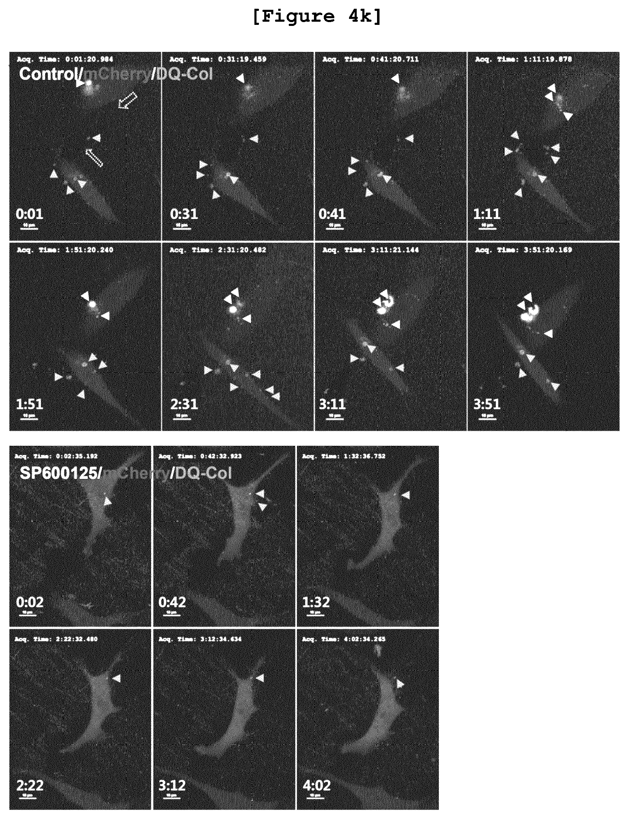

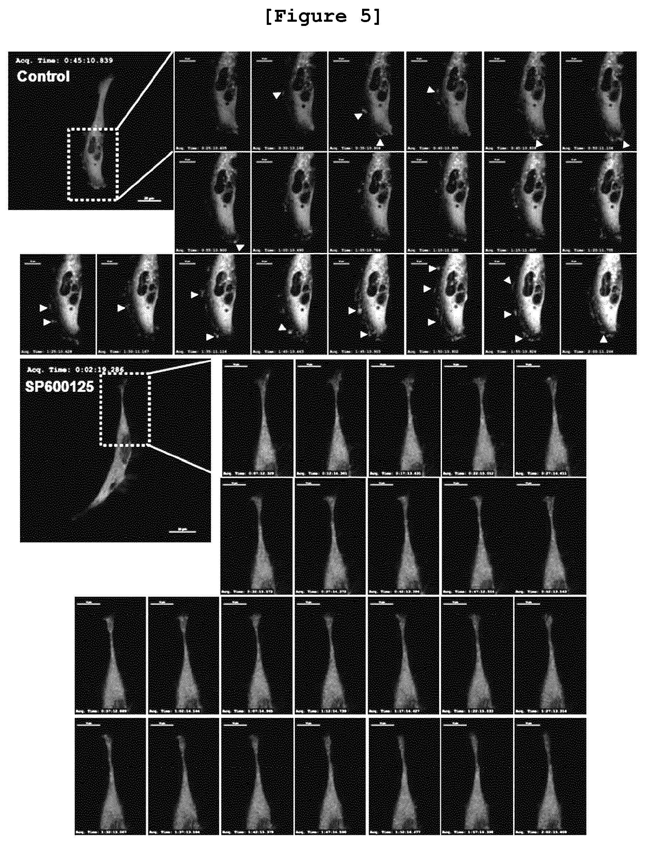

In addition, the cell shape became longer and the dynamic of the cell became weak or shrank (see Figures 2 and 4). Cell migration was reduced, and the location of cortactin was changed from cell membrane to the edge of the nucleus in cytoplasm (see Figures 5, 10, 11, and 12).

The present inventors also investigated the effect of JNK inhibitor on the formation of invadopodia in various cell lines in a three-dimensional collagen gel culture environment. Likewise, in various breast cancer cell lines, JNK inhibition changed the cell shape, increased the snail1 expression, and reduced the cortactin expression (see Figures 4E, 4F, 4G and 4J). The numbers of spots where actin and cortactin coexist were reduced (see Figure 4I), and the collagen matrix degrading activity was decreased (see Figure 4K).

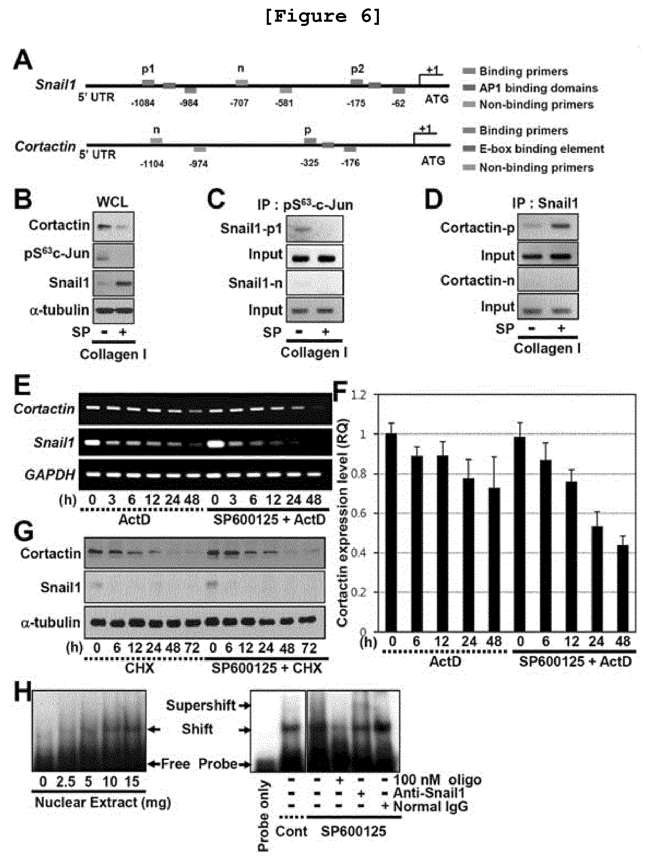

The present inventors also investigated the mechanism of the decrease of cortactin expression and the increase of snail1 expression by the treatment of JNK inhibitor in the breast cancer cell line cultured in a three-dimensional collagen gel environment, and further the inventors confirmed that JNK inhibition increased snail1 and the increased snail1 was conjugated to cortactin promoter to suppress cortactin expression (see Figures 6A ∼ 6D, and 6H). It was also confirmed that the regulation of cortactin mRNA or protein level by JNK inhibitor was controlled in the stage of transcription (see Figures 6E and 6G).

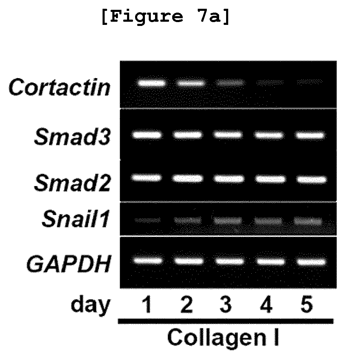

The present invention also investigated the mechanism of the increase of snail1 mRNA expression according to the treatment of JNK inhibitor in the breast cancer cell line cultured in a three-dimensional collagen gel environment. As a result, it was confirmed that JNK inhibition caused the increase of TGFβ and accordingly smad2 was up-regulated, and at the same time the phosphorylation of smad2 was also increased suggesting that snail1 was up-regulated (see Figures 7A ∼ 7E).

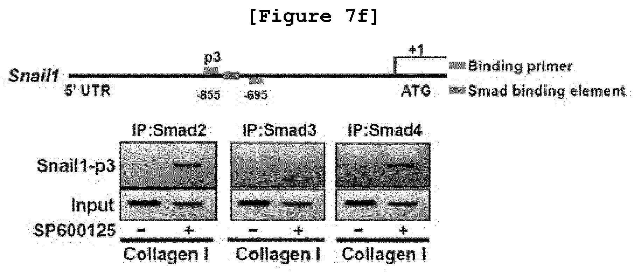

The present inventors further confirmed that when JNK inhibitor was treated to the breast cancer cell line cultured in a three-dimensional collagen gel environment, the up-regulated or activated smad protein was directly conjugated to snail1 promoter region to increase snail1 transcription (see Figure 7 F) .

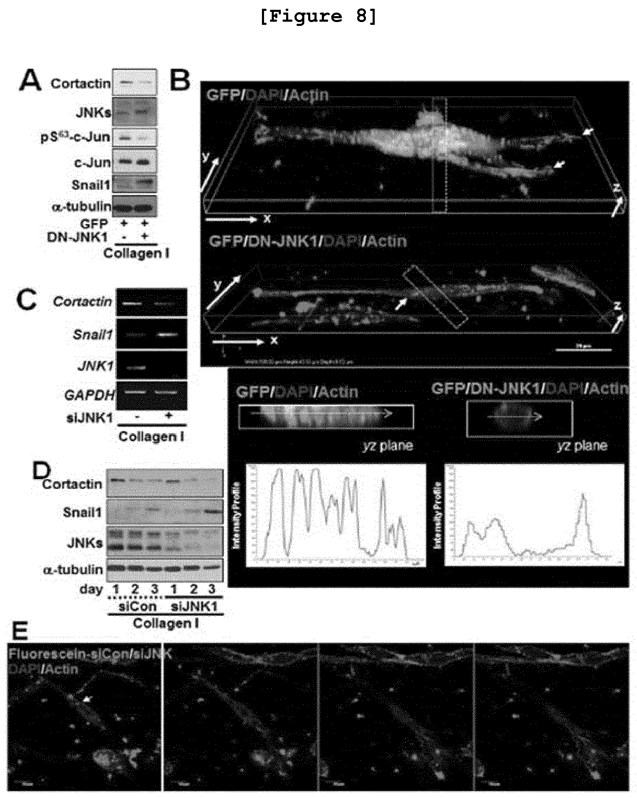

The dominant negative JNK1 was over-expressed in order to suppress JNK activity in the breast cancer cell line cultured in a three-dimensional collagen gel environment. As a result, snail1 expression was increased but cortactin expression was reduced (see Figure 8A). Also, the shape of the cells became flattened and thinner at the edge and the actin-enriched region was reduced (see Figure 8B). When JNK1 siRNA was treated to the cells, the result was consistent with those shown in the above Figure 8A and Figure 8B (see Figures 8C ∼ 8E).

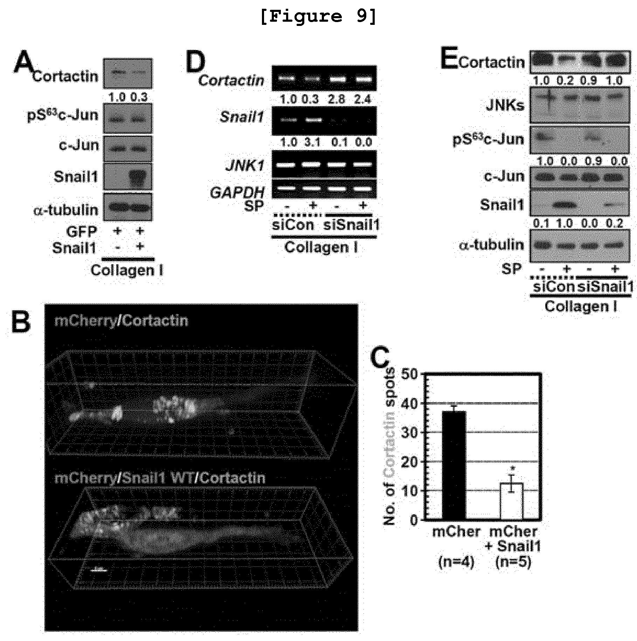

The inventors also confirmed that the expression of cortactin was decreased when snail1 was over-expressed in the breast cancer cell line cultured in a three-dimensional collagen gel environment (see Figures 9A ∼ 9C). When snail1 was knocked-down after JNK inhibition, the JNK inhibitor dependent snail1 expression was not induced anymore and the expression of cortactin was not decreased any further (see Figures 9D and 9E). Therefore, it was confirmed that snail1 expression played an important role in cortactin expression and had a negative effect on the formation of invadopodia.

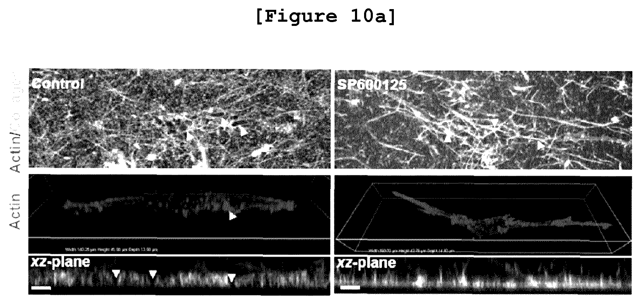

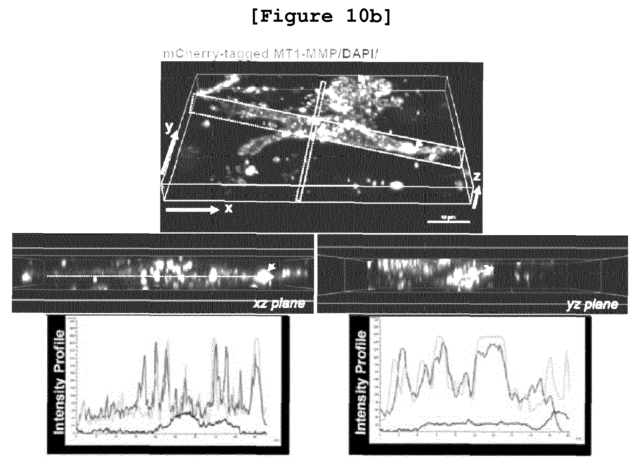

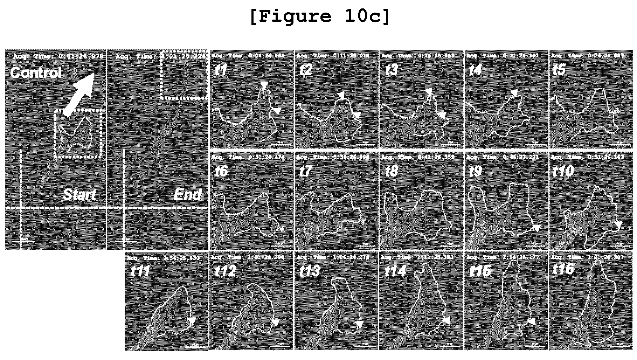

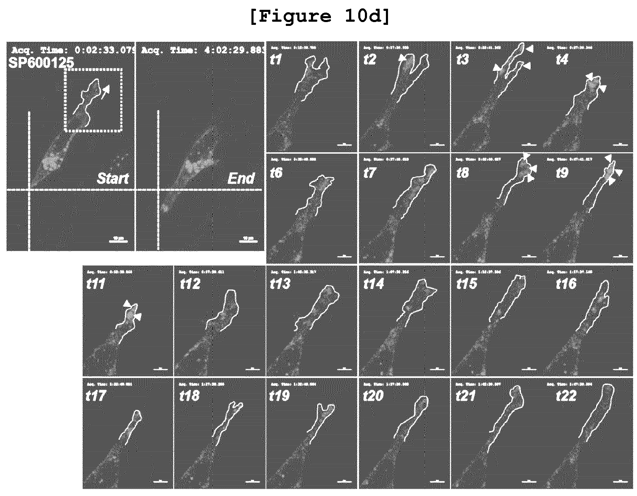

The present inventors confirmed that the degradation of collagen was observed (yellow arrow) in the breast cancer cell line cultured in a three-dimensional collagen gel environment containing green fluorescence dye-conjugated collagen, but the degradation was not observed in the cells treated with JNK inhibitor (see Figure 10A). The inventors also confirmed that MT1-MMP protein could be used as an invadopodia marker in addition to cortactin (see Figure 10B). The expression and the expression site of MT1-MMP were changed dynamically on the cell membrane starting from the edge of the cell membrane toward the moving direction of the cell (see Figure 10C). On the other hand, the expression and the expression site of the protein were not changed by the treatment of JNK inhibitor, that is the dynamic expression or inhibition of the protein was not observed and also the site was not changed (see Figure 10D). The above results indicate that MT1-MMP exists in the edge of the cell in the presence of JNK inhibitor with playing a role in cell migration and invasion processes, but cannot play any role in the degradation of ECM.

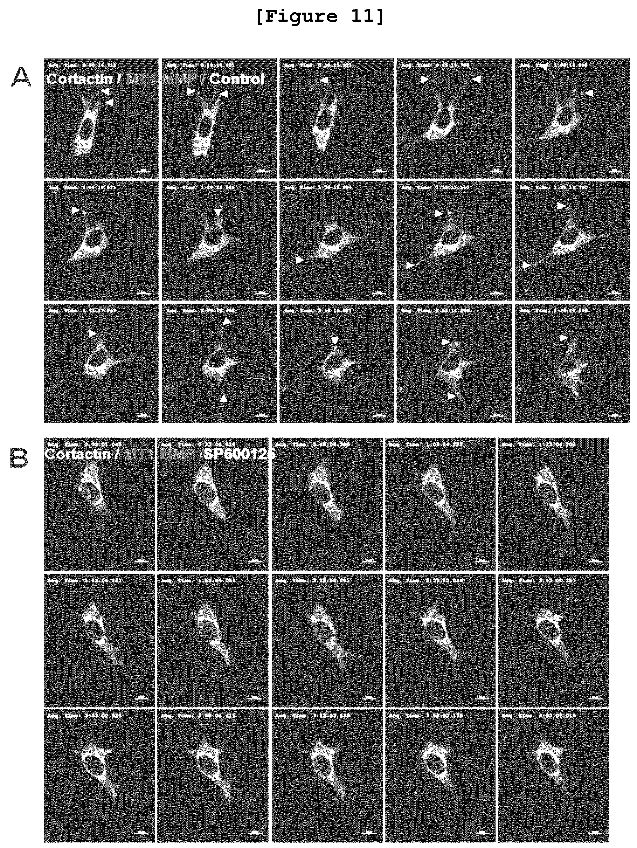

The present inventors confirmed in this invention that JNK inhibitor, when treated to the breast cancer cell line cultured in a three-dimensional collagen gel environment, changed the position of the co-expression of cortactin and MT1-MMP from the cell membrane to near the nucleus in the cytoplasm, and as a result cell invasion associated functions including ECM degradation could not be normally functioning (see Figure 11).

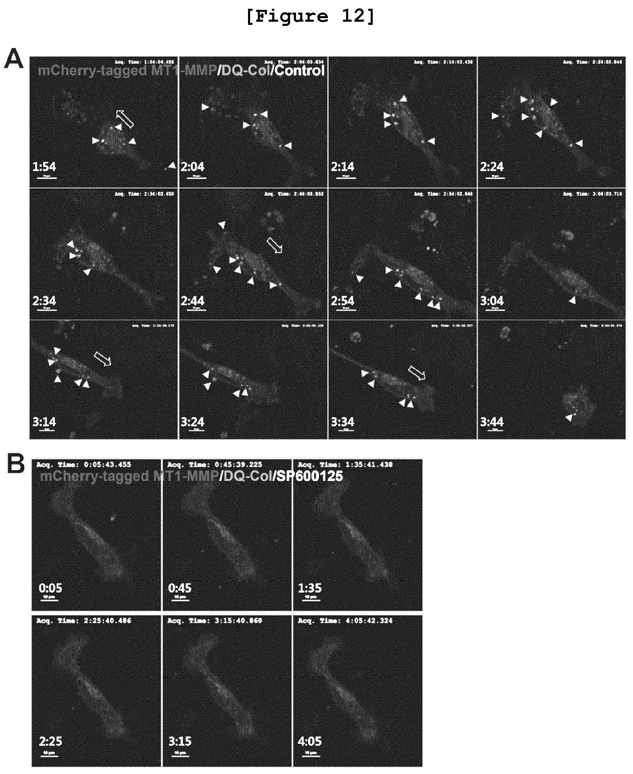

In addition, the present inventors confirmed that JNK inhibitor, when treated to the breast cancer cell line cultured in a three-dimensional collagen gel environment, suppressed MT1-MMP functions and thereby the DQ-collagen and type I collagen matrix degrading activity was decreased (see Figure 12).

Therefore, it was verified that JNK inhibition in MDA-MB-231 cell line cultured in a three-dimensional collagen gel environment caused the increase of snail1 expression but the decrease of cortactin expression, and at the same time had a negative effect on the location and role of cortactin and MT1-MMP, and as a result the formation of invadopodia and the degradation of type I collagen matrix were suppressed.

Practical and presently preferred embodiments of the present invention are illustrative as shown in the following Examples.

However, it will be appreciated that those skilled in the art, on consideration of this disclosure, may make modifications and improvements within the spirit and scope of the present invention.

### Example 1: Changes of intracellular protein caused by microenvironment in the breast cancer cell line cultured in a three-dimensional collagen gel environment

### <1-1> Preparation of polydimethylsiloxane (PDMS) culture vessel for three-dimensional cell culture

To observe the cells growing in a three-dimensional environment under confocal microscope, the PDMS culture vessel equipped with a cover glass on one side was prepared.

Particularly, PDMS crude liquid was mixed with a hardener at the ratio of 10:1, which was hardened at 100°C for 1 hour. The hardened PDMS was taken off from the mold and punched by using an 8 mm punch. A cover glass (24 x 60 mm, Marienfeld) was attached on the hole of the PDMS by treating oxygen plasma for 45 seconds, followed by drying in a 60 °C oven for 24 hours to recover hydrophobicity. The prepared PDMS was used after being irradiated with UV.

### <1-2> Culture of breast cancer cell lines in a three-dimensional collagen gel or matrigel environment

Various breast cancer cell lines were cultured in a three-dimensional type I collagen environment.

Particularly, MDA-MB-231, MDA-MB-436, MDA-MB-468, T47D, BT549, Hs578T, and MCF7 (ATCC, USA) cell lines were cultured by using PureCol type I collagen (bovine collagen I; Advanced BioMatrix, USA) or matrigel (BD Bioscience, USA). At this time, the final concentration of collagen was 2.5 ∼ 3 mg/mℓ and the concentration of matrigel was 4 ∼ 10 mg/mℓ. When the cells were treated with collagen, the strong acid collagen (pH 2) solution was adjusted to be neutral (pH 7) by using 10x reconstitution buffer [260 mM sodium bicarbonate, 250 mM HEPES, 2 N NaOH, and serum-free 10x RPMI (Sigma, USA)] so as not to induce any changes in the cell. The prepared collagen solution was stored at 4°C for 10 minutes until the collagen fibers were fully formed. To cover the bottom with collagen or matrigel in order to prevent cell adhesion on the bottom floor, 10 *µ*ℓ of the solution was poured in the PDMS vessel of 8 mm in the diameter, which stood in a 37 °C incubator for 30 minutes. While the collagen or matrigel covering the bottom floor was solidified, MDA-MB-231 cell line, which was cultured in PRMI-1640 or DMEM (JBI, Korea) supplemented with 10% FBS and penicillin/streptomycin (Invitrogen, USA), was washed with PBS twice. Then, the cells were taken off by using trypsin. The collected cells were precipitated by centrifugation, and the numbers of the cells were counted by using a hematocytometer. The cells (1 ∼ 2 x 10⁶ cells/mℓ) were well-mixed in the prepared collagen solution, which was loaded in the PDMS culture vessel covered with collagen or matrigel. The culture vessel was placed in a 37 °C incubator for 30 minutes ∼ 1 hour to harden the collagen or matrigel, followed by culture.

### <1-3> Changes in proteins in the breast cancer cell line cultured in a three-dimensional collagen gel environment

The changes of intracellular proteins in the MDA-MB-231 cell line cultured by the method of Example <1-1> were investigated by Western blotting.

Particularly, the collagen gel which was mixed with the MDA-MB-231 cell line cultured in the PDMS culture vessel by the method of Example <1-1> was collected in a microcentrifuge-tube, followed by centrifugation at 5000 rpm for 1 minute. After eliminating the supernatant, collagen gel and cell pellet were washed with cold PBS (130 mM NaCl, 13 mM Na₂HPO₄, 3.5 mM NaH₂PO₄, pH 7.4) twice, to which certain amount of lysis buffer (50 mM Tris-HCl, 150 mM NaCl, 1% NP-40 and 0.25% sodium deoxycholate) supplemented with protease inhibitor cocktails (GenDepot) was added, followed by lysis 4°C for 1 hour. The lysed sample was centrifuged at 13000 rpm for 30 minutes. The obtained supernatant was added with 4x sample buffer [200 mM Tris-HCl (pH 6.8), 8% SDS, 0.4% bromophenol blue, 40% glycerol], followed by 10 ∼ 12% SDS-PAGE. Then, the proteins were transferred onto Nitrocellulose Membranes Protran™ nitrocellulose membrane (Whatman), followed by pre-treatment with 5% skim milk. After the pre-treatment, the membrane was washed with PBS (130 mM NaCl, 13 mM Na₂HPO₄, 3.5 mM NaH₂PO₄, pH 7.4) twice, followed by reaction at 4°C for 15 hours with the mouse monoclonal antibodies of anti-E-cadherin (24E10), smad2, smad3, phospho-smad2, phospho-smad3, phospho-MAPKAPK-2 (Thr222), phospho-Ser⁶³-c-Jun, c-Jun, snail1 (L70G2) (Cell signaling, USA), cortactin, HIF1 alpha (BD bioscience, USA), slug, JNK, PCNA (Santa Cruz Biotechnology, USA), anti-MT1-MMP (Millipore, USA), and TGFβ (1, 2, 3) (R&D systems, USA). On the next day, the membrane was reacted with the secondary antibody, followed by X-ray film development by using ECL (Pierce, USA). Band intensity was measured by using Image J. Some active proteins were modified as a whole protein by α-tubulin. The relative ratios were calculated. The significance of the calculated value was examined by student's t-test, and when p-value was less than 0.05 (p-value < 0.05), it was considered as statistically significant.

As a result, as shown in Figure 1A, the phosphorylation of c-Jun and the expression of cortactin protein were reduced but the expression of snail1 was increased as the culture time became longer. However, the expressions of E-cadherin, Snail2/Slug, and c-Jun were not changed (Figure 1A). Cancer cells acquire the metastatic capability by using the microenvironment around the cell. So, it was investigated that the above result could be consistent or not when the microenvironment surrounding the cell was regulated in various aspects. As a result, when the cell density was regulated as shown in Figure 1B, or when the pH of the cell culture medium was regulated as shown in Figure 1C during 2 day-culture, or when the oxygen concentration was regulated 5 % or less as shown in Figure 1D during the culture for 4 hours, c-Jun phosphorylation and cortactin expression were reduced but snail1 expression was increased, consistently with the above results (Figures 1B ∼ 1D).

### <1-4> Protein expression in breast cancer tissue

To investigate whether or not the results of Example <1-3> could be consistent in the human breast cancer tissues, immunohistochemical staining was performed with the breast cancer patient tissues.

Particularly, the tissues obtained from 2 breast cancer patients (case #1 and case #2) were respectively fixed in 4% paraformaldehyde. The paraffin block was sliced in 4 *µ*m thickness, and the thin sections were dried to obtain paraffin sections. The paraffin-embedded tissue slide was deparaffinized and then rehydrated, followed by the treatment with 3% hydrogen peroxide for 10 minutes. One section of the slide was placed in 10 mM citrate buffer (pH 6.0), which was boiled for 20 minutes. The slide was reacted with the antibodies of pS63-c-Jun, snail1, and cortactin at 4°C for at least 18 hours. Immunohistochemical staining was performed by using streptavidin-conjugated peroxidase as the secondary antibody. Normal goat serum or normal mouse IgG showing the same subtype was used as the control. The slide section was washed with PBS (phosphate-buffered saline), to which 0.03% DAB (3,3'-diaminobenzidine tetrachloride) was added for 20 minutes, followed by observation. At the same time, counter-staining was performed with Mayer's hematoxylin, and the stained region was observed under microscope.

As a result, as shown in Figure 1E, c-Jun activity was confirmed in the invasive tumor nest region displaying metastatic capability of cancer cells and snail1 was irregularly scattered therein (Figure 1E, Case #1). However, in another cancer tissue (case #2), the expression of pS63-c-Jun in nucleus and the expression of cortactin in cytoplasm were more clearly observed in the invasive tumor edges (Figure 1E, Case #2).

Therefore, it was confirmed that the MDA-MB-231 cell line cultured in a three-dimensional collagen gel environment was affected by various microenvironments around, because of which c-Jun phosphorylation and cortactin expression were reduced but snail expression was increased.

### Example 2: Changes of cell shape and cell migration by JNK inhibitor in the breast cancer cell line cultured in a three-dimensional collagen gel environment <2-1> Changes of cell shape by JNK inhibitor

When MDA-MB-231 cell line was cultured in a three-dimensional collagen gel environment for 3 days, the volume of cytoplasm was reduced and the cell shape became comparatively thinner and longer with very dynamic end part unlike when the cell line was cultured in a two-dimensional environment. After treating the JNK inhibitor SP600125 (LC Labs), the cell shape and the migration pattern were observed.

Particularly, when the gel mixture comprising the MDA-MB-231 cell line cultured by the method of Example <1-1> and collagen was fully hardened, the culture medium supplemented with 10% FBS (control) and the culture medium supplemented with 50 µM of SP600125 (experimental group) were loaded on top of the gel, followed by culture for 3 days. Then, the shape of the cells and the migration pattern in the gel were observed under microscope.

As a result, as shown in Figures 2A and 2B, when the JNK inhibitor SP600125 was treated thereto, the shape of the cells became twice as long as the length of the control cells, and the dynamic end seen in the control cells became dull, and the contact region of the cells and the extracellular matrix became flattened and thinner as well (Figures 2A and 2B).

### <2-2> Changes in mRNA and protein expressions by JNK inhibitor

RT-PCR and Western blotting were performed to investigate the changes in mRNA and protein expressions in the MDA-MB-231 cell line cultured in a three-dimensional collagen gel environment induced by the treatment of JNK inhibitor.

Particularly, mRNA was first prepared from the total RNA obtained from the cell line treated with SP600125 as shown in Example <2-1> by using TRIzol® (Invitrogen, USA). Then, cDNA was synthesized by using AmfiRivert cDNA Synthesis Master Mix (GenDePot). PCR was performed with Thermo Scientific DreamTaq Green PCR Master Mix (Thermo Scientific). The primers used for PCR were as shown in Table 1. After PCR, electrophoresis was performed to confirm the bands on agarose gel. The band intensities were measured by using Image J. The expression level was modified by total mRNA, considering GAPDH mRNA as a standard, and then relative ratios were calculated. The significance of the calculated value was examined by student's t-test, and when p-value was less than 0.05 (p-value < 0.05), it was considered as statistically significant. To investigate the changes in protein expression, Western blotting was performed by the same manner as described in Example <1-3>.

**[Table 1]**

| Primer | Sequence | SEQ. ID. NO |
|---|---|---|
| Cortactin F | CCTGGAAATTCCTCATTGGA | 1 |
| Cortactin R | CACAAAATCAGGGTCGGTCT | 2 |
| JNK1 F | TTGGAACACCATGTCCTGAA | 3 |
| JNK1 R | ATGTACGGGTGTTGGAGAGC | 4 |
| Snail F | GGTTCTTCTGCGCTACTGCT | 5 |
| Snail R | TAGGGCTGCTGGAAGGTAAA | 6 |
| Smad2 F | CGAAATGCCACGGTAGAAAT | 7 |
| Smad2 R | CCAGAAGAGCAGCAAATTCC | 8 |
| Smad3 F | CCCCAGAGCAATATTCCAGA | 9 |
| Smad3 R | GGCTCGCAGTAGGTAACTGG | 10 |
| Twist F | GGAGTCCGCAGTCTTACGAG | 11 |
| Twist R | TCTGGAGGACCTGGTAGAGG | 12 |
| Slug F | GGGGAGAAGCCTTTTTCTTG | 13 |
| Slug R | TCCTCATGTTTGTGCAGGAG | 14 |
| GAPDH F | GAGTCAACGGATTTGGTCGT | 15 |
| GAPDH R | GACAAGCTTCCCGTTCTCAG | 16 |

As a result, as shown in Figure 2C, the levels of mRNA of snail1 and mRNA of another transcription factor Twist, known as a member of the same family with snail1, were increased as the treatment time of JNK inhibitor was longer, while the level of cortactin mRNA was reduced. In the meantime, the expression level of snail2/slug mRNA was not changed (Figure 2C). The level of snail1 mRNA did not cause any change in the expressions of E-cadherin (CDH1) mRNA and protein, suggesting that snail1 was not involved in the inhibition of E-cadherin (Figures 2C and 2D).

As shown in Figure 2D, when the cell line was treated with different concentrations of JNK inhibitor, the JNK inhibitor dependent snail up-regulation and cortactin down-regulation were observed (Figure 2D). In the meantime, JNK inhibitor did not change the phosphorylations of other MAPK proteins such as p38 and ERK (Figure 2D). The above results indicate that it is not the interaction among MAPKs but JNK that is involved in the signaling to increase snail1 expression in a three-dimensional collagen gel environment. It was also confirmed that JNK inhibition induced specifically snail1 expression.

### <2-4> Correlation between the JNK inhibitor dependent decrease of cortactin expression and the changes in cell length

To investigate whether or not the decrease of cortactin expression by JNK inhibitor was associated with the changes in cell shape being longer, cortactin protein was over-expressed in the cells treated with JNK inhibitor. Then, the shape of the cells was observed.

Particularly, the over-expression of cortactin was induced in the cell line treated with SP600125 and cultured in Example <2-1>, followed by further culture in a three-dimensional collagen gel environment for 3 days. The shape of the cells and the migration in the gel were observed under microscope.

As a result, as shown in Figure 2E, the shape of the cells that became longer by JNK inhibitor was tend to shrank by the over-expression of cortactin, suggesting that JNK inhibition caused the inhibition of cortactin expression playing an important role in cell migration/invasion as the shape of the cells became longer.

### <2-4> Inhibition of cancer cell migration and matrix degrading activity by JNK inhibitor

To confirm that the dynamic activity of the cells cultured in a three-dimensional collagen gel environment was interrupted by the JNK inhibitor SP600125 to suppress cancer cell migration and matrix degrading activity, time-lapse imaging was performed to screen the changes in cell shape and cell migration in real-time.

Particularly, when the gel mixture comprising the MDA-MB-231 cell line cultured by the method of Example <1-1> and collagen was fully hardened, the culture medium supplemented with 10% FBS (control) and the culture medium supplemented with 50 µM of SP600125 were loaded on top of the gel, followed by culture for 3 days. The culture medium was replaced every other day. The control group hardened in an incubator for 30 minutes ∼ 1 hour for time-lapse imaging and the experimental group treated with SP600125 proceeded to imaging with Olympus IX81-ZDC microscope, wherein images were obtained every 30 minutes, photo by photo, for 20 hours at 37 °C in the presence of 5% CO₂.

As a result, as shown in Figures 2F and 2G, JNK inhibition caused the significant reduction of cell migration, confirmed by the observation of a specific cell migration (Figure 2F, white arrow head). And at this time the migration route and distance were measured to make a graph. As a result, it was confirmed that there was a significant change in the migration distance (Figure 2G).

### Example 3: Effect of JNK inhibitor in the breast cancer cell line cultured in a three-dimensional matrigel environment

In this example, it was investigated whether or not the above results obtained in Example 2 were consistent with those resulted from the culture in a matrigel (another ECM) environment.

Particularly, MDA-MB-231 cell line was cultured in a three-dimensional matrigel environment treated with SP600125 and then the shape of the cells was investigated by the method of Example <2-1>. The changes in protein expression were also investigated by the method of Example <1-3>.

As a result, as shown in Figures 3A and 3B, the shape of the cells was not changed by JNK inhibitor (Figure 3A) and the expression of snail1 protein was not changed, either (Figure 3B). It was also investigated if the treatment of another MAPK, p38 or ERK inhibitor could bring the consistent results with those obtained from the treatment of JNK inhibitor. As a result, ERK inhibition did not affect cortactin expression but increased snail1 expression (Figure 3D). However, when ERK inhibitor was treated, the cells did not grow normally but instead showed dying proneness, suggesting that snail1 is involved in cell death. ERK inhibition did not reduce cortactin expression, suggesting that the result of ERK inhibition was different from that of JNK inhibition.

### Example 4: Reduction of cortactin expression by JNK inhibitor in the breast cancer cell line cultured in a three-dimensional collagen gel environment

### <4-1> Formation of invadopodia by JNK inhibitor in the breast cancer cell line cultured in a three-dimensional collagen gel environment

To investigate the changes of cell shape and migration induced by JNK inhibitor in the breast cancer cell line cultured in a three-dimensional collagen gel environment, immunofluorescence staining was performed and the formation of invadopodia was observed in the contact region of the cells and the collagen ECM on the cell membrane.

Particularly, the MDA-MB-231 cell line cultured in a three-dimensional collagen gel environment and treated with SP600125 was fixed in 4% formaldehyde (Sigma, USA) for 30 minutes. Then, the formaldehyde was eliminated and reaction was induced in 100 mM PBS glycine solution for 30 minutes, followed by permeabilization using 0.5% triton X-100 for 30 minutes. The reaction time could be adjusted in order for the cells in the collagen gel to be fully contacted with the solution. Then, the cells were pre-treated with 3% BSA solution for 2 hours. F-actin was stained with rhodamine palloidine (red) at room temperature for at least 4 hours. The cells were washed with washing buffer [0.2% triton X-100, 0.1% BSA, and 0.05% Tween 20 were added to PBS solution (pH 7.4), which was sterilized by using 0.22 *µ*m filter], and stained with Alexa488^{®}-conjugated cortactin (green) antibody at 4°C for at least 18 hours. Lastly, DAPI (4',6-diamidino-2-phenylindole; blue, Molecular Probe) staining was performed to observe the shape of nucleus. The antibody reaction time was adjusted according to the intensity of staining. The stained cells were observed under Olympus FV1000 confocal microscope and Nikon Eclipse Ti confocal microscope. Z-stack images obtained from the confocal microscope were reconstructed as 3D images by using Easy 3D modes of IMARIS software. The co-localization of the reconstructed images was analyzed by using ImarisColoc and surpass module, followed by visualization.

As a result, as shown in Figures 4A, 4B, and 4C, the sections of each channel obtained from the observation of the cells cultured in a three-dimensional collagen gel environment of the control (Figure 4A) and of the experimental group (Figure 4B) were presented in Figure 4A and Figure 4B. The 3D images obtained from the sections were reconstructed and presented in Figure 4D. The difference between the control and the experimental group, specifically section by section, was compared through fluorescence intensity profile (right bottom). To review that the result was consistent with the above, immunofluorescence staining was performed. As a result, in the cell line treated with JNK inhibitor, the shape of the cells became longer and the dynamism in there was weakened. In addition, the expressions of actin and cortactin were all reduced (Figure 4D). Matrix would be degraded in the actin-enriched spot around the cell membrane and the region of co-expression with cortactin (white arrowhead), stained as yellow. The yellow region was confirmed as invadopodia. The formation of invadopodia was suppressed by JNK inhibition, confirmed by line intensity profile shown on a specific site of XZ section. In the control, the expression site and the expression intensity of the actin-enriched spot (red) and cortactin (green) were similar. However, in the experimental group treated with JNK inhibitor, the actin-enriched spot (red) was only observed in some parts. As shown in Figure 4C, invadopodia image and graph presenting statistical analysis of invadopodia where actin coexists with cortactin were made, by which the reduction of invadopodia by JNK inhibition was quantitatively confirmed (Figure 4C).

To investigate the expression pattern of cortactin in the presence of JNK inhibitor, the cells cultured in a three-dimensional collagen gel environment were observed under confocal microscope with GFP-cortactin. Then, the changes in cortactin expression and cell morphology were photographed in a three-dimensional collagen gel environment in real-time. As shown in Figure 5, cortactin was expressed on the cell membrane of moving direction, suggesting that the expression site had been changed dynamically (white arrowhead). It was also confirmed that the treatment of JNK inhibitor caused the decrease of cell migration and the reduction of cortactin expression, suggesting that cortactin could not exist on the cell membrane (Figure 5).

### <4-2> Formation of invadopodia induced by JNK inhibitor in various breast cancer cell lines cultured in a three-dimensional collagen gel environment

To investigate whether or not the JNK inhibitor dependent formation of invadopodia in the breast cancer cell line cultured in a three-dimensional collagen gel environment was limited to human breast cancer cells or rather general phenomenon among cancers, various breast cancer cell lines, which are largely divided into four groups that display different cell morphology such as round, mass, grape-like, and stellate types when they are cultured in a three-dimensional collagen gel environment, were cultured in a three-dimensional collagen gel environment, followed by investigation of the JNK inhibitor dependent invadopodia formation. And, Western blotting was performed to investigate the changes of protein expression.

Particularly, MDA-MB-436, MDA-MB-468, MDA-MB-453, T47D, BT549, Hs578T, and MCF7 cell lines were cultured in a three-dimensional collagen gel environment treated with SP600125. Immunofluorescence staining was performed by the same manner as described in Example <4-1> and as a result the formation of invadopodia was confirmed. Western blotting was also performed by the same manner as described in Example <1-3> to measure the expression levels of intracellular proteins.

As a result, as shown in Figures 4E ∼ 4I, the changes of cell shape and the expression patterns of cortactin and snail1 induced by JNK inhibition observed in the stellate type MDA-MB-231 cell line embedded in paraffin were equally observed in the mass types T-47D and MCF-7, the grape-like type MDA-MB-468, and the stellate type MBA-MB-436 (Figures 4E and 4F). Also, the stellate type basal breast cancer cell line groups BT-549 and Hs578T displayed the longer cell shape and the reduced acin-colocalized spots (Figure 4I).

In addition, as for the intracellular protein expression, the expression of cortactin was reduced and the expression of snail1 was increased in the stellate types MDA-MB-231 and MDA-MB-436. But the expression of snail1 was not observed in the mass type and grape-like type cell lines (Figures 4E ∼ 4G). Unlike the JNK inhibition dependent snail1 expression in MDA-MB-231 and in MBA-MB-436, the expression of EMT marker such as E-cadherin, α-smooth muscle actin (α-SMA), and vimentin was not changed (Figure 4H). Therefore, it was confirmed that the basal breast cancer cell line that grew in the form of stellate in a three-dimensional environment displayed low invasiveness by pS⁶³ c-Jun, snail1, and cortactin associated signals.

### <4-3> Degradation of type I collagen by JNK inhibitor in the breast cancer cell line cultured in a three-dimensional collagen gel environment

It was investigated whether or not the type I collagen degradation was induced in the breast cancer cell line cultured in a three-dimensional collagen gel by the treatment of JNK inhibitor.

Particularly, real-time monitoring of the MDA-MB-231 cell line cultured in a three-dimensional collagen gel environment with the treatment of SP600125 was performed after treating the cells with DQ™-collagen I in order to investigate the degradation of collagen matrix in the actin-enriched spot.

As a result, as shown in Figure 4K, the collagen degradation (green spot and arrow) was confirmed in the moving direction of cells in the control, while collagen matrix was not degraded in the JNK suppressed cells (no green spot was observed) (Figure 4K).

### Example 5: Mechanism of the increase of snail1 expression and the decrease of cortactin expression by JNK inhibitor in the breast cancer cell line cultured in a three-dimensional collagen gel environment

### <5-1> Mechanism of the changes in proteins by JNK inhibitor

To investigate if the increase of snail1 expression and the decrease of cortactin expression by JNK inhibitor were attributed to the binding of the transcription factor c-Jun to the snail1 promoter region, and if the cortactin expression was regulated by the direct binding of snail1 to the cortactin promoter region, ChIP (chromatin immunoprecipitation), one of the most common *in vivo* methods to study the interaction between intracellular protein and chromatin and the activation thereof, was performed.

Particularly, the MDA-MB-231 cell line cultured in a three-dimensional collagen gel environment was fixed in 4% formaldehyde for 30 minutes. The cells were then treated with 1.5 M glycine, followed by washing with cold PBS containing protease inhibitor. The fixed cells were loaded in SDS-lysis buffer supplemented with protease/kinase inhibitor, followed by sonication for lysis. The lysed sample proceeded to electrophoresis at 13000 rpm for 3 minutes, and the supernatant was transferred into a microcentrifuge tube, followed by sonication again for chromatin fragmentation. Supernatant was obtained by centrifugation, some of which was transferred into input and some of which was reacted with the antibodies of phospho-Ser⁶³-c-Jun, snail1, and smad 2/3/4 (cell signaling technology, USA) at 4°C for at least 18 hours. Upon completion of the reaction, the tube was taken out from 4°C, to which sepharose beads coated with protein A and G (1:1), followed by reaction at 4°C for 4 hours. The tube was then washed with RIPA/150 mM NaCl solution and washed again with RIPA/350 mM NaCl solution. The washed sample was washed again with LiCl washing buffer, which was washed with TE (Tris/EDTA) solution three times. TE solution was added thereto, followed by reaction at 65°C for 15 hours. On the 3^{rd} day of the reaction, RNase A was added to each input and sample, followed by reaction at 37°C. Proteinase K was added thereto, followed by reaction at 55°C for 1 hour. Phenol/chloroform (1:1) was added thereto, followed by vortexing and then centrifugation was performed. Upon completion of the centrifugation, supernatant was collected, to which TE/200 mM NaCl and glycogen were added, followed by centrifugation, leading to the ethanol precipitation. The DNA pellet obtained from the centrifugation was naturally dried and then dissolved in sterilized distilled water. PCR was performed with the primers listed in Table 2.

**[Table 2]**

| Primer | Sequence | SEQ. ID. NO |
|---|---|---|
| Snail1 p1F | TCCAAACTCCTACGAGGC | 17 |
| Snail1 p1R | GAAGAAGTGGCAACTGCT | 18 |
| Snail1 p2F | AGCAGTTGCCACTTCTTC | 19 |
| Snail1 p2R | GCAAAGGGAAGTGTGCTT | 20 |
| Snail1 p3F | GGAGACGAGCCTCCGATT | 21 |
| Snail1 p3F | CAGTAGCGCAGAAGAACCACT | 22 |
| Snail1 pnF | CGTAAACACTGGATAAGGG | 23 |
| Snail1 pnR | GGAAACGCACATCACTGG | 24 |
| Cortactin F1 | CCTTCACATCTTGGCTAA | 25 |
| Cortactin R1 | CTAGAAGGTGAGTCAAGC | 26 |
| Cortactin F2 | GAGGGAGGATGGAGAGATGA | 27 |
| Cortactin R2 | AGAGCTCGCCCGCAACTAG | 28 |
| Cortactin F3 | TCTGCAGACTCGCCACAG | 29 |
| Cortactin R3 | CAGGCACCAGGCTCTACTTC | 30 |
| Cortactin nF | AGTGTTATGATTACAGGC | 31 |
| Cortactin nR | ATAGAGCACAGCGAAGAC | 32 |
| Smad p1F | AGCACACTTCCCTTTGCATT | 33 |
| Smad p1R | CACCCGTTCCTTCCCTTATC | 34 |
| Smad p2F | AATTTCCGCCCCCTCCCAA | 35 |
| Smad p2R | ACTCCTCCGAGGCGGGGTT | 36 |
| Smad p3F | GTCGGAAGGTCAGGTGTCC | 37 |
| Smad p3R | GACGTCGAGCGAAGCGAG | 38 |
| Smad p4F | GGAGACGAGCCTCCGATT | 39 |
| Smad p4R | CAACTCCCTTAAGTACTC | 40 |

As a result, as shown in Figures 6A ∼ 6D, when the cell line was treated with JNK inhibitor, snail1 expression was increased in the cancer cells and at the same time cortactin expression was reduced (Figure 6B). The c-Jun activated in the control was conjugated to snail1 promoter and then later the conjugation was broken because the phosphorylated c-Jun disappeared by the treatment of JNK inhibitor (Figure 6C). This result indicates that the phosphorylation of c-Jun, the downstream factor of JNK, might have an effect on snail1 transcription. When JNK inhibitor was treated, c-Jun did not bind to snail1 promoter region, so that the increase of snail1 expression induced by JNK inhibitor could be attributed not to c-Jun but to other factors (Figure 5). The inventors presumed that the JNK inhibition dependent snail1 up-regulation could regulate cortactin at the transcriptional level. So, ChIP was performed with anti-snail1 antibody. As a result, it was confirmed that the snail1 increased by JNK inhibition was conjugated to cortactin promoter (Figure 6D). According to the previous report, snail1 is conjugated to E-cadherin promoter region to inhibit E-cadherin expression and snail1 is recognized as a transcription inhibitor that suppresses gene transcription (Hemavathy K, Ashraf SI, Ip YT. Snail/slug family of repressors: slowly going into the fast lane of development and cancer. Gene. 2000;257:1-12.). Based on that, the present inventors confirmed once again that the snail1 increased by JNK inhibition was conjugated to cortactin promoter region and accordingly the expression of cortactin was suppressed.

### <5-2> Formation of snail1 and cortactin promoter protein-DNA complex induced by JNK inhibitor in the breast cancer cell line

To re-confirm the result of the above Example <5-1> which is the formation of snail1 and cortactin promoter protein-DNA complex, EMSA (electrophoretic mobility shift assay) was performed that is useful for the investigation of the size increase of DNA/protein conjugate by using ³²P-labeled probe on native acrylamide gel.

Particularly, a nuclear extract was prepared from the MDA-MB-231 cell pellet cultured in a three-dimensional collagen gel environment by using buffer C containing 50 mM HEPES pH 7.9, 50 mM KCl, 300 mM NaCl, 0.1 mM EDTA, 1 mM DTT, 0.1 mM PMSF, and 10% glycerol. Then, 10 ∼ 15 *µg* of the nuclear extract was reacted with 2 nM of ³²P-labeled probe (SEQ. ID. NO: 50; 28-mer, 5'-tagcgcttagccagctgcgggcggaccc-3') to induce snail1/probe conjugation since snail1 had been confirmed to be conjugated on the cortactin promoter region above. Thereafter, the reaction mixture was expanded on nondenaturing polyacrylamide gel at 70 V for 2 hours by using 0.5×TBE (Tris/borate/EDTA, pH 8) buffer containing 90 mM Tris base, 90 mM borate, and 0.5 mM EDTA, followed by drying for 2 hours. The isotope was exposed on X-ray film to confirm snail1-DNA complex.

For more accurate examination, an additional step of using supershift was added, wherein snail1 antibody (Cell Signaling Technology, Inc.) was additionally conjugated to protein/DNA complex before the reaction between the probe and the nuclear extract in order to reduce migration on gel or another additional step of making snail1 band weak was added, wherein 100 time higher concentration of non-labeled double-helical oligonucleotide was reacted competitively to weaken the snail1 band.

As a result, as shown in Figure 6H, the snail1 increased by JNK inhibition in the cells cultured in a three-dimensional collagen gel environment could directly bind to cortactin promoter and as a result cortactin expression was suppressed (Figure 6H).

### <5-3> Regulation of mRNA or protein level by JNK inhibitor

Western blotting, RT-PCR, and real-time PCR were performed to investigate if the result of Example <5-1> could be regulated at mRNA level or at protein level.

Particularly, new mRNA synthesis was inhibited by treating actinomycin D (ActD) suppressing transcription or new protein synthesis was inhibited by treating cyclohexamide (CHX). Then, Western blotting, RT-PCR, and real-time PCR were performed by the same manner as described in Example <1-3> and Example <2-2>. At this time, real-time PCR was performed by using SYBR Green PCR Master Mix (Applied Biosystems, USA) with 7900HT Fast real time system (Applied Biosystems, USA).

As a result, as shown in Figures 6E and 6G, the level of cortactin was significantly reduced by JNK inhibition by the treatment of ActD, confirmed by RT-PCR (Figure 6E) and real-time PCR (Figure 6F). Compared with the control, the expression of cortactin was not changed by the treatment of JNK inhibitor when new protein synthesis was inhibited by CHX (Figure 6G). The above results indicate that JNK inhibition caused the up-regulation of snail1 by regulating the stability of snail 1 or cortactin mRNA or protein, but did not cause the increase of cortactin. Therefore, it was confirmed that the reduction of cortactin observed when JNK was inhibited in the MDA-MB-231 cells cultured in a three-dimensional collagen gel environment was a result of such a mechanism that the up-regulated snail1 binds to cortactin promoter in the transcription stage so as to inhibit the transcription of cortactin and as a result cortactin is downregulated at mRNA level and further at protein level as well.

### <5-4> Regulation of snail1 mRNA or protein level by JNK inhibitor

To investigate the mechanism of increasing snail1 mRNA by JNK inhibition, the expressions of smad2 and smad3 known to regulate snail1 were confirmed at mRNA level and at protein level.

TGFβ1 pathway is induced by the conjugation between the ligand TGFβ1 and its receptors TGFβ1-receptor I and TGFβ1-receptor II. When these receptors are activated by TGFβ1, the phosphorylation and activation of smad2 or smad3 are induced, leading to the formation of a complex with smad4. Then, the complex moves into nucleus and acts as a transcription factor therein. Therefore, the present inventors measured the levels of cortactin, smad2, smad3, and snail1 mRNAs in the cells cultured for 5 days by the same manner as described in Example <2-2>. The protein expression level was also examined by the same manner as described in Example <1-3>.

As a result, as shown in Figure 7A, after 5 days of culture, the level of cortactin mRNA was reduced but the level of snail1 mRNA was increased in the cultured cells. As the culture period became longer, the expression of snail1 was reduced again (Figure 7A). However, the levels of smad2 and smad3, which were expected to regulate snail1 expression, were not changed. Therefore, it was confirmed that snail1 expression was not related to the levels of smad2 and smad3 mRNAs.

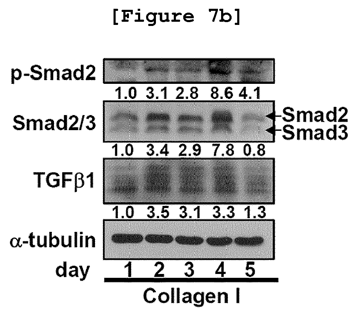

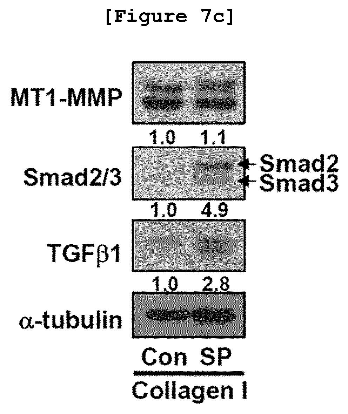

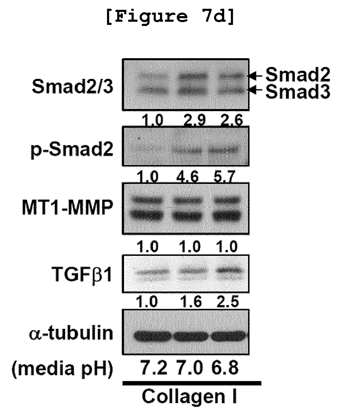

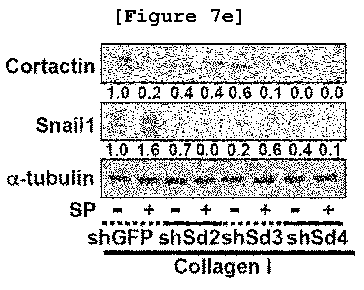

As shown in Figures 7B and 7D, it was confirmed from the examination of the protein expression that the expressions of TGFβ1, smad2 and smad3 proteins were increased slowly and caused the increase of smad2 activation (phosphorylation) while the cells were cultured for 5 days in a three-dimensional collagen gel environment during which the expression of snail1 was increasing (Figure 7B). When JNK was inhibited and extracellular pH was controlled, the results were identical to the above (Figures 7C and 7D). Therefore, it was confirmed that the expression of snail1 was associated with the changes of smad2 or smad3 protein level and the phosphorylation of smad2. As shown in Figure 7E, the experiment performed using each shRNA (short-hairpin RNA) of smad2, smad3, and smad4 supported the above results. More precisely, when the cells were treated with smad2, smad3, and smad4 shRNAs, particularly when smad2 or smad4 expression was suppressed, the phenomena induced by the treatment of JNK inhibitor such as the increase of snail1 expression and the decrease of cortactin expression were no longer observed (Figure 7E).

Therefore, it was confirmed that JNK inhibition in a three-dimensional collagen gel environment caused the increase of TGFβ1 and accordingly caused the increase of smad2 and at the same time the increase of smad2 phosphorylation together with the increase of snail1 expression.

### <5-5> Mechanism of snail1 protein up-regulation by JNK inhibitor

To investigate whether or not the increase of snail1 protein expression by smad2 and smad4 had a direct effect on the transcription level of snail, ChIP was performed by using each antibody of smad2, smad3, and smad4.

It is well informed that smad has the binding element so called "CAGA" in a three-dimensional collagen gel environment (Dennler et al., 1998). So, it was first examined that such smad binding element was there in snail1 promoter region. Then, primers were designed with the region presumed where the smad binding element was located in snail1 promoter region, followed by ChIP using the primers listed in Table 2.

As a result, as shown in Figure 7, the conjugation of smad and snail1 promoter region was only confirmed when immunoprecipitation was performed with smad 2 and smad 4 with the inhibition of JNK (Figure 7F). That is, in the MDA-MB-231 cell line cultured in a three-dimensional collagen gel environment, JNK inhibition caused the increase of smad2 and smad5 expressions and activations, and thereby the activated smad proteins directly bound to the snail1 promoter region to increase snail transcription, resulting in the increase of snail1 mRNA.

### Example 6: Reduction of invadopodia formation by the inhibition of JNK activity and the decrease of JNK protein level in the breast cancer cell line cultured in a three-dimensional collagen gel environment

Western blotting was performed by the same manner as described in Example <1-2> and immunofluorescence staining was performed by the same manner as described in Example 4 in order to investigate whether or not the snail1 expression was still as equally increased and the cortactin expression was yet decreased when JNK activity was inhibited by the over-expression of the dominant negative JNK1 in the course of examination of the cell functions in a three-dimensional collagen gel environment as when JNK inhibitor was treated to the cells.

As a result, as shown in Figures 8A and 8B, when the dominant negative JNK1 was over-expressed, the expression of snail1 was increased but the expression of cortactin was decreased (Figure 8A). As equally in the cells treated with JNK inhibitor, the shape of the cells became longer when the dominant negative JNK1 was over-expressed. The red spots in the cells indicated actin-enriched region, which seemed to be the region of invadopodia where the co-connection of matrix and cell was accomplished. As shown in the 3D image, such actin-enriched spots were observed a lot in the control, but such red spots were significantly reduced in the cells over-expressing the dominant negative JNK1, confirmed by line intensity profile. The dynamic cell shape in the edge of the cells in a three-dimensional collagen gel environment became simple by JNK1 activity inhibition (Figure 8B).

To re-confirm the above results, the experiment with JNK1 siRNA was also performed. As shown in Figures 8C and 8E, as the level of JNK1 mRNA decreased, the level of snail1 mRNA was increased but the level of cortactin mRNA was decreased (Figure 8C). When the level of JNK1 protein was reduced, the expression of cortactin was slowly decreased but the expression of snail1 was increased, compared with the control (Figure 8D). When the cells were co-transfected with GFP-conjugated control siRNA and JNK1 siRNA in a three-dimensional collagen gel environment (white arrow), the expression of JNK1 was reduced and the shape of the cells became longer and thinner and the surface of the cells seemed to be more simplified, compared with the control. In addition, the number of the actin-enriched spots (invadopodia) was also reduced, compared with the cells around which were not treated with JNK1 siRNA (Figure 8E).

### Example 7: Changes in cell shape according to the increase of snail1 expression in the breast cancer cell line cultured in a three-dimensional collagen gel environment

Western blotting was performed by the same manner as described in Example <1-3> and immunofluorescence staining was performed by the same manner as described in Example 4 in order to investigate the regulation of cortactin expression, cell shape, and actin-enriched spot population according to the regulation of snail1 expression.

As a result, as shown in Figure 9A, when snail1 was over-expressed, the level of cortactin was decreased (Figure 9A) and at this time the level of c-Jun phosphorylation was not affected by the regulation of snail1 expression, suggesting that snail1 was working as a downstream signal of c-Jun and an upstream signal of cortactin. As shown in Figures 9B and 9C, the immunofluorescence staining result proved that the over-expression of snail1 caused the decrease of cortactin expression (green spot). The invadopodia-like structure (white arrow head) was only observed in the control (Figure 9B). Compared with the control, the number of cortactin spots was significantly reduced by the increase of snail1 expression (Figure 9C). This indicates that invadopodia formation was inhibited by the snail1 increased by JNK inhibition. As shown in Figures 9D and 9E, the level of cortactin mRNA decreased by JNK inhibition was not any longer decreased when snail1 was knocked-down by using snail1 siRNA (Figure 9D). This result was also confirmed at protein level (Figure 9E). Therefore, it was confirmed that the expression of snail1 played an important role in the suppression of cortactin expression and had a great effect on the formation of invadopodia.

### Example 8: Confirmation of invadopodia marker in the breast cancer cell line cultured in a three-dimensional collagen gel environment

In invadopodia, not only cortactin but also various types of integrin and MMP (matrix metalloproteinases) proteins exist. It is known that particularly MT1-MMP (MMP14), among many MMPs, plays an important role in degrading the matrix in invadopodia. So, the inventors performed immunofluorescence staining to investigate if MT1-MMP, in addition to cortactin, could be used as another invadopodia marker in a three-dimensional collagen gel environment.

As a result, as shown in Figure 10A, the blank stained by green that suggested the collagen degradation observed in the control (yellow arrow) was not observed in the presence of JNK inhibitor (white arrow). As shown in Figure 10B, the co-localization of actin (green spot) and MT1-MMP (red spot) was confirmed (yellow spot). In membrane edge, multiple numbers of actin and MMPs were observed together (white triangle) (Figures 10A and 10B). So, it was suggested that collagen degradation could be inhibited by the treatment of JNK inhibitor in a three-dimensional collagen gel environment (Figure 10A) and MT1-MMP, in addition to cortactin, could be used as another invadopodia marker.

### Example 9: Mechanism of cell migration by the regulation of MT1-MMP expression site by JNK activation in the breast cancer cell line cultured in a three-dimensional collagen gel environment

In the presence of JNK inhibitor, how MT1-MMP, which was confirmed as another invadopodia marker, in addition to cortactin, could interact with invadopodia in the formation and dynamic was investigated.

Particularly, MDA-MB-231 cells were transfected with mCherry expression vector harboring the labeled MT1-MMP cDNA for 48 hours. Then, the cells were mixed with 2.5 mg/mℓ of type I collagen solution at the density of 10⁶ cells/mℓ. 70 *µℓ,* of the cell/collagen mixture was loaded in PDMS vessel, followed by solidification at 37 °C for 1 hour. As for the experimental group, 50 µM of SP600125, the JNK inhibitor, was treated to the medium. As for the control, the medium containing 10% FBS not treated with INK inhibitor was treated. Both were cultured for 24 hours. Any changes in dynamism of MT1-MMP expression site were traced by observing MT1-MMP location under Nikon T1 confocal microscope in real-time. At this time, to track down the location of MT1-MMP, 5 sites were selected per each sample, followed by imaging for 4 hours with taking photographs of 7 z-stacks every 5 minutes.

As a result, as shown in Figure 10C, the location of the cells was significantly changed toward the moving direction of the cells during the 4 hour-imaging in the control. Also in the control, the shape of the cell membrane was changed with diversity in the front of the cell toward the moving direction, and the dynamism of MT1-MMP expression was also confirmed, which is MT1-MMP was often located in the membrane edge and lost, reappeared and was lost again and again (Figure 10C). Such dynamic changes in cell shape and MT1-MMP expression site on cell membrane seemed to be supported by cortactin responsible for actin-branching and polymerization in cells. So, it was suggested that cell migration and invasion could be induced by such an active formation and role of invadopodia with cortactin and MT1-MMP accumulated therein, in a three-dimensional collagen gel environment. In the meantime, as shown in Figure 10D, typical MT1-MMP expression was observed without a big change in the experimental group cells treated with JNK inhibitor and the location of the cells was not changed either. Compared with the control, the number of MT1-MMP positive spots shown in the cell membrane of the front of the cell toward the moving direction was smaller but the size of the spot was much bigger, suggesting that the dynamism therein was not as much (Figure 10D). This result indicates that MT1-MMP did not take a proper spot on the cell membrane for cell migration and invasion in the presence of JNK inhibitor, and instead the spots were aggregated big and the region surrounding the nucleus became stabilized, so that MT1-MMP could not be a leading role necessary for cell migration in the front of the moving direction of the cells.

### Example 10: Changes in the cell migration pattern by the co-expression of cortactin and MT1-MMP in the breast cancer cell line cultured in a three-dimensional collagen gel environment

JNK inhibition caused the reduction of cortactin expression in the MDA-MB-231 cultured in a three-dimensional collagen gel environment and thereby directly affected cell migration and invasion, but did not affect MT1-MMP expression. To re-confirm this result, GFP-cortactin and mCherry-MT1-MMP were co-expressed, followed by treating JNK inhibitor. Then, each protein was examined to see how each protein was affected by the treatment of JNK inhibitor, by the same manner as described in Example 9.

As a result, as shown in Figure 11, MT1-MMP was repeatedly and dynamically located and lost again and again around the cell membrane of invadopodia where cortactin and MT1-MMP were co-located in the control, during which the shape of the cells became stretched long toward the moving direction of the cells (Figure 11A, white arrow head). However, as shown in Figure 11B, the spots were not observed around the cell membrane in the experimental group and the numbers of MT1-MMP spots were rather higher in the perinuclear region without any directional properties than in the cell membrane (Figure 11B). Consistently with the results of Example 9, the expression of MT1-MMP was not reduced by snail1, unlike by cortactin, but the expression location of MT1-MMP was affected by JNK inhibitor, which means that even though MT1-MMP was expressed, MT1-MMP could not be located properly on the edge of the cell membrane so that it failed to play its role in cell invasion.

Therefore, it was confirmed that JNK inhibition in the MDA-MB-231 cell line cultured in a three-dimensional collagen gel environment caused the increase of snail1 expression via TGFβ1/smad expression and signaling activity, and accordingly caused the decrease of cortactin expression, and at the same time inhibited the formation of invadopodia by negatively affecting the location and role of MT1-MMP, resulting in the inhibition of cell invasion.

### Example 11: Inhibition of type I collagen matrix degradation and suppression of MT1-MMP functions by JNK inhibitor in the breast cancer cell line cultured in a three-dimensional collagen gel environment

To investigate whether or not the reduction of MT1-MMP functions in around the cell membrane and the changes of location to the perinuclear region induced by JNK inhibition (Figures 10D and 11B) resulted in the decrease of collagen degrading activity, the collagen degrading activity of the cells expressing MT1-MMP in a three-dimensional collagen gel environment using DQ™-collagen I in real time.

Particularly, MDA-MB-231 cells were transfected with mCherry-labeled MT1-MMP or the control vector gene, followed by culture for 48 hours. 2.5 mg/mℓ of 3D collagen type I (PureCol) and 2.5 mg/mℓ of DQ™-collagen I (Life Technologies) were mixed at the ratio of 10:1 (w:w), resulting in the preparation of collagen gel, in which the cultured cells were embedded at the density of 1.5×10⁶ cells/ml. Then, the gel was solidified at 37°C for 30 minutes, on which 10% FBS/RPMI-1640 medium supplemented with 200 mℓ of DMSO or 50 mM SP600125 was added, followed by further culture. 2 ∼ 4 hours after embedding, imaging of the cells was performed by Nikon eclipse Ti (Nikon Plan-Apochromat 60×/1.4 N.A) confocal microscope for 4 ∼ 6 hours in total with taking 5 photographs every 10 minutes with 0.7 µm z-stack.

As a result, as shown in Figure 12, the mCherry labeled (red) MT1-MMP and the degraded type I collagen (green) were observed in base and cell body region in the control (Figure 12A; blue arrow). However, when JNK was suppressed by SP600125, the degradation of collagen I was not observed. This result indicates that JNK inhibition could suppress the matrix degrading activity of MT1-MMP (Figure 12B).

Those skilled in the art will appreciate that the conceptions and specific embodiments disclosed in the foregoing description may be readily utilized as a basis for modifying or designing other embodiments for carrying out the same purposes of the present invention. Those skilled in the art will also appreciate that such equivalent embodiments do not depart from the spirit and scope of the invention as set forth in the appended Claims.

## Claims

1. A method for monitoring cancer cell migration, invasion, metastasis, and the degree of metastasis, comprising the following step:
1) culturing cancer cells in a culture vessel in a three-dimensional environment surrounded by extracellular matrix; and
2) measuring the changes in the shape of cancer cells cultured in step 1), and the activity, expression, and changes in expression sites of the proteins associated with invadopodia formation/degradation, migration, invasion, and metastasis, and the degradation of extracellular matrix.

2. The method for monitoring cancer cell migration, invasion, metastasis, and the degree of metastasis according to claim 1, wherein the cell culture in step 1) is performed under the regulation of cell culture period, cell number (density), extracellular pH, or extracellular oxygen level.

3. The method for monitoring cancer cell migration, invasion, metastasis, and the degree of metastasis according to claim 1, wherein the cancer of step 1) is a metastatic cancer or a metastasis inducible cancer.

4. The method for monitoring cancer cell migration, invasion, metastasis, and the degree of metastasis according to claim 3, wherein the metastatic cancer is selected from the group consisting of breast cancer, liver cancer, stomach cancer, colon cancer, bone cancer, pancreatic cancer, head/neck cancer, uterine cancer, ovarian cancer, rectal cancer, esophageal cancer, small bowel neoplasm, anal cancer, colon carcinoma, fallopian tube carcinoma, endometrial carcinoma, uterine cervical carcinoma, vaginal carcinoma, vulva carcinoma, Hodgkin's disease, prostatic cancer, bladder cancer, kidney cancer, ureter cancer, renal cell carcinoma, renal pelvic cancer, and central nervous system tumor.

5. The method for monitoring cancer cell migration, invasion, metastasis, and the degree of metastasis according to claim 1, wherein the culture vessel of step 1) is made of one of those materials selected from the group consisting of polydimethylsiloxane (PDMS), polymethylmethacrylate (PMMA), polyacrylates, polycarbonates, polycyclic olefins, polyimides, and polyurethanes.

6. The method for monitoring cancer cell migration, invasion, metastasis, and the degree of metastasis according to claim 1, wherein the three-dimensional culture environment is selected from the group consisting of laminin, collagen, fibronectin, and hyaluronic acid.

7. The method for monitoring cancer cell migration, invasion, metastasis, and the degree of metastasis according to claim 6, wherein the collagen is type I collagen.

8. The method for monitoring cancer cell migration, invasion, metastasis, and the degree of metastasis according to claim 6, wherein the collagen is included at the concentration of 1 ∼ 5 mg/mℓ.

9. The method for monitoring cancer cell migration, invasion, metastasis, and the degree of metastasis according to claim 1, wherein the change in the shape in step 2) is **characterized by** being longer and the contact region of the cells and the extracellular matrix became flattened and thinner.

10. The method for monitoring cancer cell migration, invasion, metastasis, and the degree of metastasis according to claim 1, wherein the change in the shape in step 2) is used for the confirmation of invadopodia formation recognized by the expression of actin, cortactin, or MT1-MMP or by the co-expression thereof.

11. The method for monitoring cancer cell migration, invasion, metastasis, and the degree of metastasis according to claim 1, wherein the cell migration and invasion of step 2) are confirmed by investigating the moving distance, direction and speed of the cells.

12. The method for monitoring cancer cell migration, invasion, metastasis, and the degree of metastasis according to claim 1, wherein the change in the protein activity in step 2) is **characterized by** the increase of c-Jun phosphorylation.

13. The method for monitoring cancer cell migration, invasion, metastasis, and the degree of metastasis according to claim 1, wherein the change in the protein activity in step 2) is **characterized by** the changes in the phosphorylations and expressions of TGFβ1, smad2, and smad3 caused by JNK activation or c-Jun phosphorylation.

14. The method for monitoring cancer cell migration, invasion, metastasis, and the degree of metastasis according to claim 1, wherein the change in the protein expression in step 2) is **characterized by** the increase of snail1 transcription resulted from the binding of c-Jun to snail1 promoter region.

15. The method for monitoring cancer cell migration, invasion, metastasis, and the degree of metastasis according to claim 1, wherein the change in the protein expression in step 2) is **characterized by** the decrease of cortactin transcription resulted from the binding of snail1 to cortactin promoter region.

16. The method for monitoring cancer cell migration, invasion, metastasis, and the degree of metastasis according to claim 1, wherein the change in the expression site of the protein in step 2) is **characterized by** the increased expression of either cortactin or MT1-MMP not in plasma membrane but in cytoplasm and perinuclear region.

17. The method for monitoring cancer cell migration, invasion, metastasis, and the degree of metastasis according to claim 1, wherein the measurement of the activity, expression, and changes in expression sites of the proteins is performed by the method selected from the group consisting of Western blotting, RT-PCR, real-time PCR, immunofluorescence, ChIP (chromatin immunoprecipitation), EMSA (Electrophoric Mobility Shift Assay), and ECM degrading activity assay using DQ™-collagen type I.

18. A method for screening a cancer metastasis inhibitor comprising the following steps:
1) culturing cancer cells in a culture vessel in a three-dimensional environment surrounded by extracellular matrix;
2) treating test samples to the cancer cells of step 1);
3) measuring the activity, expression, and changes in expression sites of the proteins associated with invadopodia formation/degradation, migration, invasion, and metastasis, and the degradation of extracellular matrix; and
4) selecting the test sample that is confirmed to inhibit the formation of invadopodia or inhibit the activity and expression of the invadopodia marker protein or the metastasis associated protein or to have the negative effect on the expression sites of those proteins or on the degradation of extracellular matrix.

19. The method for screening a cancer metastasis inhibitor according to claim 18, wherein the cancer of step 1) is a metastatic cancer or a metastasis inducible cancer.

20. The method for screening a cancer metastasis inhibitor according to claim 18, wherein the metastasis associated protein of step 3) is selected from the group consisting of JNK, c-Jun, TGFβ1, smad2, smad3, cortactin, snail1, and MT1-MMP.

21. The method for screening a cancer metastasis inhibitor according to claim 18, wherein the change in the expression site of the metastasis associated protein in step 3) is **characterized by** the increased expression of either cortactin or MT1-MMP not in plasma membrane but in cytoplasm and perinuclear region.

22. The method for screening a cancer metastasis inhibitor according to claim 18, wherein the measurement of the activity, expression, and changes in expression sites of the proteins in step 3) is performed by the method selected from the group consisting of Western blotting, RT-PCR, real-time PCR, immunofluorescence, ChIP (chromatin immunoprecipitation), EMSA (Electrophoric Mobility Shift Assay), and ECM degrading activity assay using DQ™-collagen type I.

23. The method for monitoring cancer cell migration, invasion, metastasis, and the degree of metastasis according to claim 1, wherein the measurement of the migration and invasion of step 2) is to confirm the collagen gel matrix degrading activity.

24. The method for screening a cancer metastasis inhibitor according to claim 18, wherein the measurement of the migration and invasion of step 3) is to confirm the collagen gel matrix degrading activity.


### Sequence Listing

<110> Medicinal Bioconvergence Research Center
<120> Monitering method for metastasis of cancer using cancer cell cultured in 3-dimensional collagen gels environments
<130> 13P-04-25
<150> KR 10-2013-0054262
   <151> 2013-05-14
<160> 40
<170> KopatentIn 1.71
<210> 1
   <211> 20
   <212> DNA
   <213> Artificial Sequence
<220>
   <223> Cortactin F
<400> 1
   cctggaaatt cctcattgga 20
<210> 2
   <211> 20
   <212> DNA
   <213> Artificial Sequence
<220>
   <223> Cortactin R
<400> 2
   cacaaaatca gggtcggtct 20
<210> 3
   <211> 20
   <212> DNA
   <213> Artificial Sequence
<220>
   <223> JNK1 F
<400>
   ttggaacacc atgtcctgaa 20
<210> 4
   <211> 20
   <212> DNA
   <213> Artificial Sequence
<220>
   <223> JNK1 R
<400> 4
   atgtacgggt gttggagagc 20
<210> 5
   <211> 20
   <212> DNA
   <213> Artificial Sequence
<220>
   <223> Snail F
<400> 5
   ggttcttctg cgctactgct 20
<210> 6
   <211> 20
   <212> DNA
   <213> Artificial Sequence
<220>
   <223> Snail R
<400> 6
   tagggctgct ggaaggtaaa 20
<210> 7
   <211> 20
   <212> DNA
   <213> Artificial Sequence
<220>
   <223> Smad2 F
<400> 7
   cgaaatgcca cggtagaaat 20
<210> 8
   <211> 20
   <212> DNA
   <213> Artificial Sequence
<220>
   <223> Smad2 R
<400> 8
   ccagaagagc agcaaattcc 20
<210> 9
   <211> 20
   <212> DNA
   <213> Artificial Sequence
<220>
   <223> Smad3 F
<400> 9
   ccccagagca atattccaga 20
<210> 10
   <211> 20
   <212> DNA
   <213> Artificial Sequence
<220>
   <223> Smad3 R
<400> 10
   ggctcgcagt aggtaactgg 20
<210> 11
   <211> 20
   <212> DNA
   <213> Artificial Sequence
<220>
   <223> Twist F
<400> 11
   ggagtccgca gtcttacgag 20
<210> 12
   <211> 20
   <212> DNA
   <213> Artificial Sequence
<220>
   <223> Twist R
<400> 12
   tctggaggac ctggtagagg 20
<210> 13
   <211> 20
   <212> DNA
   <213> Artificial Sequence
<220>
   <223> Slug F
<400> 13
   ggggagaagc ctttttcttg 20
<210> 14
   <211> 20
   <212> DNA
   <213> Artificial Sequence
<220>
   <223> Slug R
<400> 14
   tcctcatgtt tgtgcaggag 20
<210> 15
   <211> 20
   <212> DNA
   <213> Artificial Sequence
<220>
   <223> GAPDH F
<400> > 15
   gagtcaacgg atttggtcgt 20
<210> 16
   <211> 20
   <212> DNA
   <213> Artificial Sequence
<220>
   <223> GAPDH R
<400> 16
   gacaagcttc ccgttctcag 20
<210> 17
   <211> 18
   <212> DNA
   <213> Artificial Sequence
<220>
   <223> Snail1 p1F
<400> 17
   tccaaactcc tacgaggc 18
<210> 18
   <211> 18
   <212> DNA
   <213> Artificial Sequence
<220>
   <223> Snail1 p1R
<400> 18
   gaagaagtgg caactgct 18
<210> 19
   <211> 18
   <212> DNA
   <213> Artificial Sequence
<220>
   <223> Snaill p2F
<400> 19
   agcagttgcc acttcttc 18
<210> 20
   <211> 18
   <212> DNA
   <213> Artificial Sequence
<220>
   <223> Snail1 p2R
<400> 20
   gcaaagggaa gtgtgctt 18
<210> 21
   <211> 18
   <212> DNA
   <213> Artificial Sequence
<220>
   <223> Snail1 p3F
<400> 21
   ggagacgagc ctccgatt 18
<210> 22
   <211> 21
   <212> DNA
   <213> Artificial Sequence
<220>
   <223> Snail1 p3F
<400> 22
   cagtagcgca gaagaaccac t 21
<210> 23
   <211> 19
   <212> DNA
   <213> Artificial Sequence
<220>
   <223> Snail1 pnF
<400> 23
   cgtaaacact ggataaggg 19
<210> 24
   <211> 18
   <212> DNA
   <213> Artificial Sequence
<220>
   <223> Snail1 pnR
<400> 24
   ggaaacgcac atcactgg 18
<210> 25
   <211> 18
   <212> DNA
   <213> Artificial Sequence
<220>
   <223> Cortactin F1
<400> 25
   ccttcacatc ttggctaa 18
<210> 26
   <211> 18
   <212> DNA
   <213> Artificial Sequence
<220>
   <223> Cortactin R1
<400> 26
   ctagaaggtg agtcaagc 18
<210> 27
   <211> 20
   <212> DNA
   <213> Artificial Sequence
<220>
   <223> Cortactin F2
<400> 27
   gagggaggat ggagagatga 20
<210> 28
   <211> 19
   <212> DNA
   <213> Artificial Sequence
<220>
   <223> Cortactin R2
<400> 28
   agagctcgcc cgcaactag 19
<210> 29
   <211> 18
   <212> DNA
   <213> Artificial Sequence
<220>
   <223> Cortactin F3
<400> 29
   tctgcagact cgccacag 18
<210> 30
   <211> 20
   <212> DNA
   <213> Artificial Sequence
<220>
   <223> Cortactin R3
<400> 30
   caggcaccag gctctacttc 20
<210> 31
   <211> 18
   <212> DNA
   <213> Artificial Sequence
<220>
   <223> Cortactin nF
<400> 31
   agtgttatga ttacaggc 18
<210> 32
   <211> 18
   <212> DNA
   <213> Artificial Sequence
<220>
   <223> Cortactin nR
<400> 32
   atagagcaca gcgaagac 18
<210> 33
   <211> 20
   <212> DNA
   <213> Artificial Sequence
<220>
   <223> Smad p1F
<400> 33
   agcacacttc cctttgcatt 20
<210> 34
   <211> 20
   <212> DNA
   <213> Artificial Sequence
<220>
   <223> Smad p1R
<400> 34
   cacccgttcc ttcccttatc 20
<210> 35
   <211> 19
   <212> DNA
   <213> Artificial Sequence
<220>
   <223> Smad p2F
<400> 35
   aatttccgcc ccctcccaa 19
<210> 36
   <211> 19
   <212> DNA
   <213> Artificial Sequence
<220>
   <223> Smad p2R
<400> 36
   actcctccga ggcggggtt 19
<210> 37
   <211> 19
   <212> DNA
   <213> Artificial Sequence
<220>
   <223> Smad p3F
<400> 37
   gtcggaaggt caggtgtcc 19
<210> 38
   <211> 18
   <212> DNA
   <213> Artificial Sequence
<220>
   <223> Smad p3R
<400> 38
   gacgtcgagc gaagcgag 18
<210> 39
   <211> 18
   <212> DNA
   <213> Artificial Sequence
<220>
   <223> Smad p4F
<400> 39
   ggagacgagc ctccgatt 18
<210> 40
   <211> 18
   <212> DNA
   <213> Artificial Sequence
<220>
   <223> Smad p4R
<400> 40
   caactccctt aagtactc 18